# EUROPEAN PATENT SPECIFICATION

(11) **EP 2 474 624 B1**
(45) Date of publication and mention of the grant of the patent: **17.08.2016**
(21) Application number: 11150229.0
(22) Date of filing: 05.01.2011
(51) Int. Cl.: C12Q 1/68, G01N 33/574

(54) **Response prediction in cancer treatment (p53 adapted cancer therapy)**
Reaktionsvorhersage bei der Krebsbehandlung (p53-adaptierte Krebstherapie)
Prédiction de la réponse dans le traitement du cancer (thérapie du cancer adaptée p53)

(43) Date of publication of application: 11.07.2012
(73) Proprietor: Kandioler, Daniela, 3032 Eichgraben (AT)
(72) Inventor: Kandioler, Daniela, 3032 Eichgraben (AT)
(74) Representative: Sonn & Partner Patentanwälte

(56) References cited:
- WO-A1-2005/065723
- TOKALOV SERGEY V ET AL: "Protection of p53 wild type cells from taxol by nutlin-3 in the combined lung cancer treatment", BMC CANCER, vol. 10, no. 1, 23 February 2010 (2010-02-23), page 57, XP021066938, BIOMED CENTRAL, LONDON, GB ISSN: 1471-2407, DOI: 10.1186/1471-2407-10-57
- KAPPEL S ET AL: "Turning the tables on surgical oncology: The - Pancho trial unplugged", EUROPEAN SURGERY - ACTA CHIRURGICA AUSTRIACA, vol. 40, no. 6, 2008, pages 277-283, XP002633761, ISSN: 1682-8631, DOI: 10.1007/S10353-008-0438-5
- KANDIOLER D ET AL: "Growing clinical evidence for the interaction of the p53 genotype and response to induction chemotherapy in advanced non-small cell lung cancer", JOURNAL OF THORACIC AND CARDIOVASCULAR SURGERY, vol. 135, no. 5, 1 May 2008 (2008-05-01), pages 1036-1041, XP22631731, ST. LOUIS, MO, US ISSN: 0022-5223, DOI: 10.1016/J.JTCVS.2007.10.072 [retrieved on 2008-05-01]
- Kandioler D., et al.: "A prospective study of the interaction between p53 genotype and overall survival in patients with colorectal cancer liver metastases (CRCLM) with and without neoadjuvant therapy (oxaliplatin and cape) (Abstr.n° e15003).", J Clin Oncol ASCO Annual Meeting Abstracts, vol. 27, no. 15s May 2009 (2009-05), XP002633762, Retrieved from the Internet: URL:http://www.asco.org/ASCOv2/Meetings/Ab stracts?&vmview=abst_detail_view&confID=65 &abstractID=35285 [retrieved on 2011-04-20]
- Kandioler D. et al: "p53 adapted neoadjuvant therapy for esophageal cancer: Pilot study. (Abstr. n° 4535)", J Clin Oncol ASCO Annual Meeting Abstracts, vol. 25, no. 18s 20 June 2007 (2007-06-20), XP002633763, Retrieved from the Internet: URL:http://www.asco.org/ASCOv2/Meetings/Ab stracts?&vmview=abst_detail_view&confID=47 &abstractID=32925 [retrieved on 2011-04-20]
- KANDIOLER-ECKERSBERGER D ET AL: "TP53 mutation and p53 overexpression for prediction of response to neoadjuvant treatment in breast cancer patients.", CLINICAL CANCER RESEARCH : AN OFFICIAL JOURNAL OF THE AMERICAN ASSOCIATION FOR CANCER RESEARCH, vol. 6, no. 1, January 2000 (2000-01), pages 50-56, XP002633764, ISSN: 1078-0432
- KANDIOLER: "Personalized medecine-p53 gene analysis for prediction of response to neoadjuvant therapy in esophageal cancer", MEMO, vol. 1, 2008, pages 137-142, XP002633765, DOI: 10.1007/s12254-008-0049-7

## Description

The invention relates to the field of tumour therapy.

Tumour diseases (or cancers (cancer diseases), i.e. malignant neoplasms) is a class of diseases in which a group of cells displays uncontrolled growth (division beyond the normal limits), invasion (intrusion on and destruction of adjacent tissues), and sometimes metastasis (spread to other locations in the body via lymph or blood). These three malignant properties of cancers differentiate them from benign tumours, which are self-limited, and do not invade or metastasise. Most cancers form a (solid) tumour mass but some, like leukemia, do not.

Tumour diseases affect people at all ages with the risk for most types increasing with age. Such diseases cause a rising number of deaths; in most countries between 10 and 30 % of all human deaths.

Tumour diseases are caused by abnormalities in the genetic material of the transformed cells. These abnormalities may be due to the effects of carcinogens, such as tobacco smoke, radiation, chemicals, or infectious agents. Other tumour disease-promoting genetic abnormalities may randomly occur through errors in DNA replication, or are inherited, and thus present in all cells from birth. The heritability of tumour diseases is usually affected by complex interactions between carcinogens and the host's genome.

Therapy of a tumour disease (also referred to as: cancer management options) is currently performed in many ways, the most important being chemotherapy, radiation therapy, surgery, immunotherapy and monoclonal antibody therapy. The choice of therapy depends upon the location and stage of the tumour and the grade of the disease, as well as the general state of a person's health. Experimental cancer treatments are also under development. It is also common to combine more than one therapy for the treatment of a tumour patient.

Complete removal of the tumour without damage to the rest of the body is the goal of treatment. Sometimes this can be accomplished by surgery, but the propensity of the tumour disease to invade adjacent tissue or to spread to distant sites by microscopic metastasis often limits its effectiveness. Surgery often required the removal of a wide surgical margin or a free margin. The width of the free margin depends on the type of the cancer, the organ affected, and the method of removal. The effectiveness of chemotherapy is often limited by toxicity to other tissues in the body. Radiation can also cause damage to normal tissue.

When describing effects of treatment regimens it is important to determine the direction of between-treatment difference among patient's subsets. Effects from qualitative and quantitative interaction have to be distinguished (Gail et al., Biometrics 41 (1985), 361-372).

A quantitative interaction occurs if the treatment effect varies in magnitude but not in direction across all patient subgroups. It is frequently referred to as non-crossover interaction and leads to a therapy effect in responders and no effect in non-responders. So in some patients the therapy may not help, but does not do harm either.

In case of a qualitative interaction (crossover interaction) the between-treatment difference changes direction among patient subsets. This means that the application of a certain therapy improves outcome of some patients (responder) and has an inverse effect on other patients. A qualitative interaction is the strongest interaction known between treatment and patient outcome and creates great differences between groups (Gail et al., 1985).

A qualitative interaction profoundly influences patient outcome and trial outcome if not realised.

The concept of qualitative interaction is statistically known (Gail et al., 1985). A qualitative interaction between a marker and treatment outcome has not yet been described in cancer therapy. The proof of a qualitative interaction generates demand for marker testing due to ethical and safety considerations.

Tokalov et al. (BMC CANCER 10 (2010), 57) report protection of p53 wild type cells from taxol by nutlin-3 in the combined lung cancer treatment; Kappel et al. (SUR. 40 (2008), 277-283) investigate how p53 genotype affects chemotherapy treatment in esophageal cancer; Kandioler et al. (J.THOR.CARDIO.VASC.SURG. 135 (2008), 1036-1041) disclose clinical evidence for the interaction of the p53 genotype and response to induction of chemotherapy in advanced non-small cell lung cancer; Kandioler et al. (J.CLIN.ONCOL. 27 (2009), Abstract Nr. e15003) disclose results of a prospective study of the interaction between p53 genotype and overall survival in patients with colorectal liver metastases (CRCLM) with and without neoadjuvant therapy; Kandioler et al. (J.CLIN.ONCOL. 25 (2007), Abstract Nr. 4535) report about a p53 adapted neoadjuvant therapy for esophageal cancer; Kandioler-Eckersberger et al. (CLIN.CAN.RES. 6 (2000), 50-56) disclose Tp53 mutation and p53 overexpression for prediction of response to neoadjuvant treatment in breast cancer patients; Kandioler (MEMO 1 (2008), 137-142) describes p53 gene analysis for predicition of response to neoadjuvant therapy in esophageal cancer; WO 2005/065723 A1 discloses screening methods for functional p53.

It is an object of the present invention to improve effectiveness of treatment of tumour diseases. A specific object is the prevention of worsening the status of a cancer patient by choosing the wrong treatment strategy, i.e. to prevent a negative effect of a tumour therapy which is (although being effective in some patients) harming the patient being treated with a treatment not being appropriate for the tumour.

Therefore, the present invention provides a method as defined in claims 1 to 3.

Disclosed is also a method for diagnosing a tumour patient
(i) whether the tumour patient should be treated with a therapy inducing p53 dependent apoptosis or should be treated with a therapy interfering with the cell cycle and/or
(ii) whether the tumour patient must not be treated with a therapy inducing p53 dependent apoptosis or must not be treated with a therapy interfering with the cell cycle characterised by the following steps:
   - determining the genetic status of the patient's tumour with respect to p53 by determining in a sample of body fluid or a tissue sample of the patient containing tumour cells or cell-free tumour DNA whether the whole p53 gene is present in native form or whether the p53 gene has one or more mutations;
   - diagnosing the tumour patient as

   - (i) a patient who should be treated with a therapy inducing p53 dependent apoptosis if the whole p53 gene is present in native form or as a patient who should be treated with a therapy interfering with the cell cycle if the p53 gene has one or more mutations; and/or
   - (ii) a patient who must not be treated with a therapy inducing p53 dependent apoptosis if the p53 gene has one or more mutations or as a patient who must not be treated with a therapy interfering with the cell cycle if the whole p53 gene is present in native form.

The present invention is based on the identification of a qualitative interaction between the marker p53 and response to the treatment of the tumour disease.

The p53 tumour suppressor is a 393-aa transcription factor. In response to various types of genotoxic stresses, p53 transactivates a number of genes by binding to specific DNA sequences, thereby arresting cell cycle, repairing damaged DNA, or inducing apoptosis as the cell fates. The structure of the p53 core DNA-binding domain (residues 94-312) that binds directly to the DNA sequence has been resolved by x-ray crystallography, and both x-ray crystallography and NMR analysis have been used to deduce the structure of the tetramerisation domain (residues 323-356), which is needed for optimum function. The structure of the p53 Protein is composed of 6 functional domains. The amino-terminal residues one to 42 and 43 to 63 contain two transactivation domains. The first one can be bound by MDM2, a negative regulator of p53 and the second one can bind to p53-responsive elements in promoters of different p53-regulated genes to activate their transcription. The proline-rich domain spanning residues 61-94 is involved in apoptosis and protein-protein interactions. The largest domain including residues 102-292 functions in binding p53-responsive sequences associated with genes regulated by p53. The p53 protein functions as tetramer. Tetramerization is accomplished by residues 324-355. The carboxy-terminal domain from residue 363 to 393 regulates the stability and DNA binding activity of the p53 protein (reviewed by. Belyi et al., Cold Soring Harbor Perspectives in Biology 2010;2:a001198). The p53 activity is regulated by posttranslational mechanisms such as phosphorylation, methylation, acetylation, and prolyl-isomerisation, or by protein-protein interaction, thereby it becomes stabilised and can conduct its respective physiological function (reviewed by Olsson et al., Cell death and Differentiation 14 (2007), 1561-1575).

Somatic TP53 mutations are the most common (about 50%) genetic alteration in human cancer, and a large number of TP53 mutations have been assembled in TP53 mutation databases. The latest International Agency for Research on Cancer (IARC R14 from 2009) TP53 mutation database contains 26597 somatic mutations and 535 germ-line mutations. Among these, 89-8% of p53 mutations are clustered in the core DNA-binding domain and over 70% of the mutations are missense mutations. So far, 4235 distinct mutations including 1586 amino acid substitutions caused by missense mutations have been documented. Compared to all mutations published, there is little increase in the number of newly described mutations since the year 2002 (10% per year) and even less increase in the number of newly described amino acid substitutions (2.85% per year) (IARC p53 mutation database release R14 from November 2009; Petitjean et al. *mutant p53 functional properties on TP53 mutation patterns and tumor phenotype:* Hum Mutat. 28 (2007), 622-629). Therefore it seems that most tumour associated missense mutations have been already identified and unreported missense mutations might be non-pathogenic for tumour development. Furthermore 2314 p53 mutants representing all possible amino acid substitutions caused by a point mutation throughout the coding sequence have been evaluated using a functional assay (Kato et al., PNAS 100 (2003), 8424-8429).

Due to its important role in tumour biology, p53 has been in the focus of tumour diagnosis, and especially as a potential predictive marker for therapy response.

The initial observation that p53 accumulation might serve as a surrogate biomarker for TP53 mutation has been the cornerstone for vast translational efforts aimed at validating its clinical use for the diagnosis, prognosis, and treatment of cancer. Early on, it was realised that accurate evaluation of p53 status and function could not be achieved through protein-expression analysis only. As the understanding of the p53 pathway has evolved and more sophisticated methods for assessment of p53 functional integrity have become available, the clinical and molecular epidemiological implications of p53 abnormalities in cancers are being revealed. They include diagnostic testing for germline and somatic p53 mutations, and the assessment of selected p53 mutations as biomarkers of carcinogen exposure and cancer risk and prognosis. The strengths and limitations of the most frequently used techniques for determination of p53 status in tumours, as well as the most remarkable latest findings relating to its clinical and epidemiological value are described most recently by Robles et al. (CSH Perspect. Biol. 2 (2010), a001016). The most important methodologies for assessment of p53 status in clinical and epidemiological studies are DNA sequencing, immuno-histochemistry (IHC), TP53 mutational load assay, mass spectrometry, microarray analysis and functional analysis (reviewed by Robles et al., 2010).

DNA sequencing is an established method for identification of TP53 mutations and often the method of choice for such purpose. Gel-based mutation screening assays, such SSCP or PCR-RFLP, are routinely used before sequencing. In this technique, TP53 is amplified and resulting PCR fragments are subjected to enzymatic restriction using an enzyme for which a site is predicted to be created or destroyed by the presence of mutation. The resulting gel profile after enzymatic restriction or due to denaturising conditions is used as an indicator for the presence of mutation, and sequencing of that area is undertaken using direct sequencing methods. Most TP53 mutations identified in tumours are circumscribed to the area encompassing exons 5-8 and therefore many translational studies have limited their mutational analysis to this portion of the gene. This has caused bias in the TP53 mutation literature because mutations outside exons 5-8 may have been missed. Additionally mutations may have been missed due to the limited sensitivity of the screening techniques. The problem is likely to be solved with more sophisticated targeted high-throughput DNA sequencing strategies that may in the future be used to gather nucleotide-level information about TP53 and other critical genes in clinical samples. Still, the presence of mutation does not unequivocally indicate that p53 is fully inactive, nor does the absence of it indicate that p53 is functionally proficient. Thus, assessing functional activity of p53 mutants was regarded as essential for an accurate indication of clinical relevance (Robles et al., 2010).

Most of the currently applied anticancer therapies use one of two different pathways to attack cancer cells: They either act via apoptosis or interfere with the cell cycle. p53 is crucially involved in both pathways:
Pathway 1: cancer therapy induces DNA damage and subsequent apoptosis: DNA damage is a strong trigger for p53 activation. Activated p53 transactivates apoptosis genes which lead to cell death. This mechanism has been suggested e.g. for drugs acting as antimetabolites, antibiotics or alkylating agents (not for drugs acting in the M phase).
Pathway 2: cancer therapy interferes with (different phases of) the cell cycle: In case of p53 mutation, cells cannot be controlled (arrested) in G1 phase of the cell cycle. Cells are cycling unbreakable. Therefore more cells are in S or M phase which makes them sensitive for cell cycle interfering drugs (synchronization effect).

In case of p53 the marker identifies a patient subset (s a) which will not be treated successfully but will be harmed by a certain type of therapy. At the same time the p53 status of the tumour determines potentially effective therapies (other pathway) for this subset of patients. It follows that normal p53 enhances the activity of apoptosis inducing cancer therapy but impairs activity of cell cycle interfering agents; it also follows that mutant p53 enhances activity of cell cycle interfering cancer therapy but impairs activity of apoptosis inducing agents.

| | | |
|---|---|---|
| | Pathway 1 | Pathway 2 |
| | Apoptosis | Cell cycle |
| p53 normal | enhance | impair |
| p53 mutant | impair | enhance |

Table 1: Association between p53 status and response to cancer therapy.

The p53 status of a tumour determines which type of therapy will be successful but also which therapy will harm the patient. The qualitative interaction describes the variation in direction of the therapy effect (one therapy is superior for some subsets and the alternative treatment is superior for the other subsets). Additionally there is an inverse magnitude of effect (= wrong treatment harms patients which can be seen in survival curves showing worse outcome for patients receiving a "non p53 adapted therapy" when compared to those receiving no treatment (i.e. in case of non p53 adapted therapy the treatment does not help but harms).

With the present invention it is safeguarded that the tumour patient receives the appropriate treatment and - even more important - is protected from suffering the negative impact of the wrong treatment.

The prevention of the negative impact of the wrong tumour treatment with respect to the action of p53 or p53 mutants has never been considered at all, because the qualitative interaction has not been recognised; quite in contrast, it is a central dogma of current treatment practice for tumour diseases that combinations of treatments are applied, even though added effectiveness was not affirmed for such combination. However, current practice turned out to be wrong according to the present invention: The present invention aims at preventing the negative consequences of a non p53 adapted treatment. With the present invention the new teaching is used that combining substances of both pathways mentioned above does not only mean that one substance is not effective but that this substance causes side effects and harms the patients or even prevents the positive effects of the other drug or treatment.

The present invention allows not only the selection of patients who will not respond to a certain therapy (a small number of such markers is currently used, such as Her2neu, oestrogen-receptor, kras), but also determines active therapies for those patients (suggesting the use of drugs belonging to the other pathway). Drugs which will harm the patient can be identified as well as drugs which will not be helpful (or even be harmful either) in a combination therapy.

According to the present invention, a drug is defined as being active or inactive and harmful based on their mode of action and on the genotype of the marker p53.

As p53 is the most commonly mutated gene in human cancer, the concept according to the present invention is applicable to almost all tumour-types and is valid for all anticancer drugs which interfere in some way with apoptosis or cell cycle, at least for those tumour types where p53 connected apoptosis has relevance for chemotherapy or where p53 mutations impair normal apoptosis function of p53.

A critical review of the data from literature in view of the present invention with respect to the qualitative p53-therapy-interaction reveals surprising results: In contrast to the present believe that a combination of more than one type of tumour disease treatment (i.e. a drug acting via the apoptosis route in combination with a drug which acts on the cell cycle) is acceptable, even if there is no proven benefit for such combination, it becomes clear with the present invention that combining substances of both pathways means that one substance is not only not effective but causes side effects and harms the patient.

The literature is full of clinical trials using combinations of drugs "with non-synergistic pathways", as far as it concerns p53. The moderate response improvement resulting from the introduction of combination treatments in cancer therapy can be explained as follows: those patients who did not respond to the drug of the first pathway (due to p53 mutation) could have benefited from the substance of the second pathway. However, the benefit of the second drug is moderate, because due to the added side effects both drugs have to be delivered in a reduced dose. Therefore the effective drug cannot show is full potential in such combinations.

The teachings of the present invention can therefore be used for explaining numerous studies wherein antitumour drugs or antitumour treatments have been applied with contradicting results. For example, De Laurentiis et al. (J. Clin. Oncol. 26 (2008), 44-53) have reported a taxane based combination as adjuvant chemotherapy of early breast cancer as a meta analysis of randomised trials. It was disclosed that combination therapies require dose-reduction for both compounds, but may, in theory, exploit drug synergism. With the teachings of the present invention it is clear that this essentially depends on whether drugs for the same pathway have been applied or not. On the other hand, De Laurentiis also concluded that in sequential regimens, both compounds can be administered at optimal doses. The crucial issue (whether taxanes should be combined with anthracyclines (or whether they should be administered after an anthracycline-based regimen)) could not be answered; in this metaanalysis, "only sequential regimens yielded a statistically significant improvement of both DFS and OS". This observation can be explained by the teaching of the present invention: as in these trials always drugs addressing different pathways have been applied (taxanes/anthracyclines) a positive effect was only possible if substances were applied sequentially (because then the potentially positive effect of one substance is not affected by the negative effect of the other )).

Francis et al. (J. Natl. Cancer Inst. 100 (2008), 121-133) report on adjuvant chemotherapy with sequential or concurrent anthracycline and docetaxel (breast International Group 02-98 Randomised trial). It was concluded that important differences may be related to doxorubicin and docetaxel scheduling, with sequential but not concurrent administration, appearing to produce better DFS than anthracycline based chemotherapy. Both papers (De Laurentiis et al. and Francis et al.) came to the same conclusion - that sequential but not concurrent administration produces better results - but they had no idea why. The present invention does explain this effect and turns these scientific discoveries into a new breakthrough regime for the treatment of tumour diseases, showing that a sequential administration is not necessary at all (because only one drug is actually effective).

The present invention also explains why the numerous retrospective studies, evaluating p53 as a predictive marker, produced inconsistent results so far: trials which used (without recognizing) drug combinations from both pathways in their treatment regimen may have acted differentially with p53 than trials which used drug combinations from only one pathway or monotreatment. Therefore the trial results are inconsistent and the power of p53 predicting response could not be demonstrated so far.

From the statistical point of view, this phenomenon has been described by Gail et al. (1985) : "It is possible to have highly significant qualitative interactions without a significant overall effect."

However, in contrast to being a mere explanation of mechanism, the present invention provides the teaching that there are "wrong" treatments of tumour diseases which significantly harm the patient. From this teaching it is clear that defining the p53 status of a patient's tumour before deciding about the nature of tumour treatment(s) is essential. Therefore, the present invention provides a tumour treatment which essentially requires the definition of the p53 status of a patient's tumour and then the administration of a "p53 status suitable" antitumour drug and - and this is a significant part of the present invention - the prevention of administration of an antitumour drug which is not "p53 status suitable". An antitumour drug which is "p53 status suitable" is an apoptosis inducing drug for patients with p53 normal tumours and a cell cycle interfering drug for patients with a p53 mutant tumour status; an antitumour drug which is not "p53 status suitable" is an apoptosis inducing drug for patients with a p53 mutant tumour status and a cell cycle interfering drug for patients with a p53 normal tumour.

Preferably, the p53 status of a patient's tumour is defined by a sample of body fluid or a tissue sample of the patient which is a blood sample or a tumour biopsy sample (containing histologically verified tumour cells). This is based on the reliable determination of the genetic p53 status of a given tumour cell of a tumour patient which requires the provision of a sample of tumour cells of this patient. Such a sample can be a tissue specimen, e.g. a tumour biopsy or a suspension of tumour cells harvested by any method, or a sample of a body fluid from such a patient, such as blood (or a blood derived sample, such as serum or plasma), cerebrospinal fluid, lymph, ascitic fluid, or any other body-derived liquid containing tumour cells. The "tumour status" of such cells has to be verified first either by histological or biochemical (immunological) or genetic verification or other means of verification.

The present invention is applicable for all types of tumour diseases, i.e. for all cancer patients.

Accordingly, preferred tumour diseases according to the present invention are solid tumours, especially colorectal cancer, esophagus cancer, gallbladder cancer, lung cancer, breast cancer, oral cancer, ovarian cancer, pancreas cancer, rectal cancer, gastrointestinal cancer, stomach cancer, liver cancer, kidney cancer, head and neck cancer, cancer of the nervous system, retinal cancer, non-small cell lung cancer, brain cancer, soft tissue cancer, lymphnode cancer, cancer of the endocrine glands, bone cancer, cervix cancer, prostate cancer or skin cancer; or a haematological tumour, preferably acquired aplastic anaemia, myelodysplastic syndrome, acute myeloid leukaemia, acute lymphatic leukaemia, Hodgkin lymphoma, non-Hodgkin lymphoma or multiple myeloma.

An important aspect of the present invention is to demonstrate and preserve the power of the marker p53 by providing and claiming a highly sensitive testing method and therewith preserving the marker for clinical use and application in tumour patients.

As already stated above, an important aspect of the present invention is to prevent the "wrong" treatment for a tumour patient. Accordingly, the present invention relates to a method for predicting negative consequences of a treatment of a tumour patient with a therapy inducing p53 dependent apoptosis or a therapy interfering with the cell cycle which is characterised by the following steps:
- determining the genetic status of the patient's tumour with respect to p53 by determining in a sample of body fluid or a tissue sample of the patient containing tumour cells or cell-free tumour DNA whether the whole p53 gene is present in native form or whether the p53 gene has one or more mutations;
- predicting the tumour patient as

- (i) a patient who will suffer negative consequences of a therapy interfering with the cell cycle if the whole p53 gene is present in native form; or
- (ii) a patient who will suffer negative consequences of a therapy inducing p53 dependent apoptosis if the p53 gene has one or more mutations.

In a similar manner, a method for predicting an enhanced treatment effect of a treatment of a tumour patient with a therapy inducing p53 dependent apoptosis or a therapy interfering with the cell cycle is disclosed which is characterised by the following steps:
- determining the genetic status of the patient's tumour with respect to p53 by determining in a sample of body fluid or a tissue sample of the patient containing tumour cells or cell-free tumour DNA whether the whole p53 gene is present in native form or whether the p53 gene has one or more mutations;
- predicting the tumour patient as

- (i) a patient who will expect an enhanced treatment effect of a therapy inducing p53 dependent apoptosis if the whole p53 gene is present in native form; or
- (ii) a patient who will expect an enhanced treatment effect of a therapy interfering with the cell cycle if the p53 gene has one or more mutations.

The teachings of the present invention enable a significantly improved treatment of tumour patients. Whereas up to now only a small number of markers for a specific treatment was used in very isolated manner: Her2neu, for example is overexpressed in only 20 to 25 % of breast cancer patients; treatment with trastuzumab, a humanised monoclonal antibody against HER2/neu, increased survival (6% vs. 8.5%), less recurrence and less metastases (Viani et al., BMC Cancer 7 (153) (2007)). These improvements are, however, significantly less as the improvements according to the present invention. This allows an improved method for treatment of a tumour patient which is characterised by the following steps:
- determining the genetic status of the patient's tumour with respect to p53 by determining in a sample of body fluid or a tissue sample of the patient containing tumour cells or cell-free tumour DNA whether the whole p53 gene is present in native form or whether the p53 gene has one or more mutations;
- treating the tumour patient

- (i) with a therapy inducing p53 dependent apoptosis if the whole p53 gene is present in native form and avoiding any therapy interfering with the cell cycle; or
- (ii) with a therapy interfering with the cell cycle if the p53 gene has one or more mutations and avoiding any therapy inducing p53 dependent apoptosis.

Almost all antitumour drugs nowadays applied can be grouped in one of the groups according to the present invention (apoptosis/cell cycle). At least for those drugs, the present invention fully applies.

Therefore, the present invention can be applied for the following apoptosis inducing therapies:
- a treatment with methotrexate, 5-fluorouracil, capecitabine, gemcitabine or hydroxyurea;
- a treatment with actinomycin D or anthracyclins, especially doxorubicin, daunorubicin, idarubicin, valrubicin, mitoxantrone or epirubicin;
- a treatment with melphalan, oxazaphosphorins, especially cyclophosphamide, ifosfamide or busulfan; nitrosourea, especially carmustine, lomustine, semustine or procarbazine; or a treatment with cisplatin, carboplatin or oxaliplatin;
- a treatment with thymidylate synthase inhibitors, especially raltitrexed or pemetrexed;
- radiotherapy;
- a treatment with estrogens, gestagens, anti-estrogens, especially tamoxifen, 3-hydroxy-tamoxifen, or chlortamoxifen; aromatase inhibitors, especially aminoglutethimide, formestan, anastrozol or letrozol; antiandrogens, especially cyproterone acetate or flutamide; gonadotropin-releasing hormone antagonists (buserelin, goserelin, leuprolerin, triptorelin); or
- a treatment with antitumour antibodies, preferably a treatment with a HER2 inhibitor antibody, especially trastuzumab; with a EGFR inhibitor antibody, especially cetuximab, panitumumab or nimotuzumab; with a thymidine kinase inhibitor antibody, especially gefitinib or erlotinib.

As well the present invention is applied for the following therapies which interfere with the cell cycle:
- a treatment with camptothecins, especially irinotecan or topotecan;
- a treatment with vinca alcaloids, especially vincristine, vinblastine, vindesine or vinorelbine; or a treatment with taxanes, especially paclitaxel or docetaxel.

Also combinations with two or more treatments is possible, provided that the treatments belong to the same group, i.e. either from the apoptosis inducing group or from the cell cycle interfering group.

There are several important conclusions which can be drawn from the concept according to the present invention for cancer therapy:
- It does not make sense to combine substances of the two different pathways (which is, however, very common today; accordingly, here the present invention is a significant change in paradigm).
- It is useful to go for maximal synergy regarding the p53 pathway between chemotherapeutic agents.
- In case of a mutant p53 status patients should be spared from apoptosis inducing therapies and radiation therapy.
- In case of a normal p53 status patients should not receive cell cycle interfering drugs.
- Any new substance should be tested for its place in the p53 interaction model to avoid combination of drugs using different pathways.
- Based on the strong interaction and the frequency of mutations in almost all tumour types, the p53 status of a tumour has to be addressed in clinical trials as a stratification criterion. Otherwise the true potential of a drug can not be assessed.

As used herein, the terms "cancer" and "tumour disease" are drawn to identical subject matter for the present application; the tumour diseases and the patients with tumour diseases according to the present invention are cancers and cancer patients which are or have malignant tumours, respectively. Accordingly, the tumour patients according to the present invention are not patients having benign tumours.

According to another aspect, a method for treatment of a tumour patient is disclosed which is characterised by the following steps:
- determining the genetic status of the patient's tumour with respect to p53 by determining in a sample of body fluid or a tissue sample of the patient containing tumour cells whether the whole p53 gene is present in native form or whether the p53 gene has one or more mutations;
- treating the tumour patient

- (i) with a therapy inducing p53 dependent apoptosis if the whole p53 gene is present in native form and avoiding any therapy interfering with the cell cycle; or
- (ii) with a therapy interfering with the cell cycle if the p53 gene has one or more mutations and avoiding any therapy inducing p53 dependent apoptosis, especially avoiding chemotherapy and radiation.

As a preferred embodiment of this method wherein a patient is treated with a therapy interfering with the cell cycle (ii), the method further comprises a treatment of said patient with a drug inducing a cell cycle arrest in normal cells in said patient before said therapy interfering with the cell cycle. This can be any drug applied with the intention to induce a p53 dependent reversible, cytoprotective cell cycle arrest in p53 normal cells while p53 mutant tumour cells are treated with a cell cycle interfering drug. Preferred examples of such drugs are nutlin or actinomycin D (Dactinomycin, Cosmegen oder Lyovac-Cosmegen). In case of mutant p53 genotype of a tumour advantage of the normal p53 status of the normal cells can be taken: In normal cells a reversible cell cycle arrest can be induced using a systemic drug (preferably nutlin or actinomycin D). These drugs activate normal p53 and subsequently induce reversible cell cycle arrest. Cell cycle arrest is therefore restricted to p53 normal cells and has a cytoprotective effect on them while mutant cells can be effectively treated with a cell cycle interfering drug. Normal cells resting in a reversible cell cycle arrest will not be affected by the cell cycle interfering drug and side effects will be prevented.

A p53 mutation according to the present invention is defined as any mutation in the genetic set-up of the tumour cell which affects the primary amino acid sequence of p53 protein and decreases apoptosis induction activity of the p53. It follows that the p53 mutations according to the present invention are all mutations resulting in frameshifts and all deletions and insertions in the coding region. Moreover, all single base substitutions in the coding area which result in a change in primary amino acid sequence are p53 mutations according to the present invention as well as mutations in the regulating regions which cause loss or decreased expression of p53 in comparison to healthy tissue. Finally, all mutations affecting splice sites, thereby resulting in a p53 protein with different amino acid sequence, are also included.

Genetic polymorphisms, i.e. variants which are present in normal tissue too, and silent mutations, i.e. mutations which cause no change in the encoded amino acid sequence, are, of course, not defined as p53 mutations according to the present invention.

Examples for p53 mutations according to the present invention are disclosed e.g. in Kato et al., PNAS 100 (2003), 8424-8429. Other examples can be found in various databases for p53, such as the IARC (International Agency for Research on Cancer; somatic p53 mutations in neoplastic cells or tissues, including metastases or cells derived from such cells or tissue).

The results of DNA sequencing in the course of the present invention, which is currently the most reliable method for p53 mutation analysis, comprise changes of nucleotides. For evaluation of the functional consequences caused by mutations several approaches have been proposed in the scientific literature as outlined below.

Based on the location of the mutation and the predicted amino acid alterations two categories of p53 mutations have been defined by Poeta et al. (N. Engl. J. Med. 357 (2007), 2552-2561): Disruptive mutations are non-conservative changes of amino acids located inside the key DNA-binding domain (L2-L3 region), or all DNA sequence alterations that introduce a STOP codon resulting in disruption of p53 protein production; non-disruptive mutations are conservative changes of amino acids (replacement of an amino acid with another from the same polarity/charge category) or non-conservative mutations outside the L2-L3 region (excluding stop codons).

As reviewed by Joerger et al. (Oncogene 26 (2007), 2226-2242) p53 mutations lead to a variety of structural and energetic changes in the protein. Recently, using molecular modelling Carlsson et al. (FEBS J. 276 (2009), 4142-4155) proposed a stability measure of the mutated p53 structure to predict the severity of mutations. Taking structural features and sequence properties into account a classification into deleterious and non-deleterious mutations was performed.

The functional property of mutant p53 proteins may also be represented by their transactivation activities (TAs), as measured in eight p53 response elements in yeast assays by Kato et al. (2003) and expressed as a percentage of wildtype protein.

The TAs for all possible missense mutations obtained by single-nucleotide substitution along the full coding sequence of p53 are listed in the database of the International Agency for Research on Cancer (Petitjean et al., Hum. Mutat. 28 (2007), 622-628). Perrone et al proposed the median TAs to be calculated and mutations to be classified as fully functional (median TA > 75% and < 140%), partially functional (median TA > 20% and ≤ 75%), or non-functional (median TA ≤ 20%) (Perrone et al., J.Clin. Oncol. 28 (2010), 761-766).

All these approaches are based on the detection of nucleotide changes (mutations) brought together with general knowledge of protein expression (mechanisms of translation) as well as the functional domains of the protein. Thereby the impact on the function of the protein can be deduced. A base exchange at a certain position may create a stop codon, lead to the usage of another amino acid at this position or produce no apparent alteration. These changes happen at the level of translation, but a base exchange may be already effective in mRNA processing. A translationally silent mutation may produce or disrupt a splice site as well as a binding site for regulatory factors (proteins, microRNAs). On the other hand, even in case of an amino-acid exchange a protein may retain some of its normal functions.

Based on these arguments all mutations qualify as functionally relevant in some way unless there is a comprehensive scientific proof of normal function in-vivo.

Preferred p53 mutations to be detected according to the present invention are all mutations in the p53 gene, especially
- all mutations resulting in frameshifts
- all deletions and insertions in the coding region
- all single base substitutions in the coding area which result in a stop codon (nonsense mutations)
- all single base substitutions proven to affect splice sites in vivo or in vitro

Of the single base substitutions resulting in a change of the primary amino acid sequence or potentially affecting splice sites the following have been directly tested for interaction with chemotherapy in clinical studies:

**Table 2**

| p53 Mutation | Pathway |
|---|---|
| c.313G>T (p.Gly105Cys) | 1 |
| c.332T>A (p.Leu111Gln) | 2 |
| c.380C>T (p.Ser127Phe) | 1 |
| c.422G>A (p.Cys141Tyr) | 1 |
| c.452C>G (p.Pro151Arg) | 1 |
| c.461G>T (p.Gly154Val) | 1 |
| c.467G>C (p.Arg156Pro) | 2 |
| c.473G>A (p.Arg158His) | 1 |
| c.488A>G (p.Tyr163Cys) | 1 |
| c.524G>A (p.Arg175His) | 1/ 2 |
| c.578A>T (p.His193Leu) | 1 |
| c.584T>C (p.Ile195Thr) | 1 |
| c.641A>G (p.His214Arg) | 2 |
| c.653T>A (p.Val218Glu) | 1 |
| c.659A>G (p.Tyr220Cys) | 1 |
| c.707A>G (p.Tyr236Cys) | 1 |
| c.711G>C (p.Met237Ile) | 1 |
| c.733G>A (p.G1y245Ser) | 1/ 2 |
| c.742C>T (p.Arg248Trp) | 1 |
| c.743G>A (p.Arg248Gln) | 1 |
| c.743G>T (p.Arg248Leu) | 1 |
| c.746G>T (p.Arg249Met) | 1 |
| c.747G>T (p.Arg249Ser) | 1 |
| c.749C>T (p.Pro250Leu) | 2 |
| c.785G>T (p.Gly262Val) | 1 |
| c.794T>C (p.Leu265Pro) | 1 |
| c.811G>A (p.Glu271Lys) | 1 |
| c.817C>T (p.Arg273Cys) | 1 |
| c.818G>A (p.Arg273His) | 1 |
| c.818G>T (p.Arg273Leu) | 1 |
| c.821T>C (p.Val274Ala) | 1 |
| c.824G>A (p.Cys275Tyr) | 1 |
| c.827C>A (p.Ala276Asp) | 1 |
| c.833C>G (p.Pro278Arg) | 1 |
| c.833C>T (p.Pro278Leu) | 1 |
| c.844C>T (p.Arg282Trp) | 1/ 2 |
| c.1025G>C (p.Arg342Pro) | 1 |
| c.919+1G>T | 2 |
| c.994-1G>A | 1 |

Table 2: Single base substitutions detected in tumours and evaluated for interaction with cancer therapy according to pathway 1 (apoptosis induction) or pathway 2 (cell-cycle interference). For patients with tumours bearing one of the mutations listed, the therapy regimen acting in pathway 1 were not effective, those in pathway 2 led to improved outcome.

If any of these mutations occur in the analysis of the tumour cells according to the present invention, the p53 status is "mutated". Preferably, the presence/absence of the mutations according to Table 2 are investigated by the method according to the present invention (these can also be tested for a tumour already diagnosed in principle)

In summary currently the issue of functional evaluation of p53 mutations has not been finally addressed, the proposed classifications arose from basic research approaches (partly in vitro) and are not proven in appropriately designed clinical trials.

Appropriately designed clinical trials currently do not exist because the clinical evaluation of the utility of p53 mutations deserves a number of considerations listed as follows:
1. The treatments used in such trials have to be synergistic considering their pathway of action or have to be single drugs (monotherapy).
   The qualitative interaction and the two pathway model has not yet been realised. A combination of treatments from different pathways - which is very common today- will bias the trial results. Consequently appropriate clinical trials currently do not exist.
2. The selection of the appropriate patient population and the choice of an adequately measurable end point.
   Ideally, the population studied should be one in which the knowledge of the marker would have substantial clinical relevance (e.g. tumour type with a high frequency of p53 mutations) and where the feasibility of obtaining appropriate specimens is established.
3. The adequate endpoint in a trial testing for therapeutic interaction is response to treatment. Measurement of response to treatment requires a clinical setting which allows a correct (pathological) response assessment. Pathological response assessment is available only for patients who are treated having their tumours in place. (i.e. preoperatively (neoadjuvantly) treated patients or patients treated for metastases).
4. The use of qualified statistical designs to allow statistical test for interaction. The latter requires specification of subsets (p53 normal, p53 mutant) in advance. For identification of the subsets (p53 normal, p53 mutants) a reliable method has to be used.
5. The issues listed above cannot be fulfilled retrospectively and therefore an evaluation of the clinical utility of p53 as a predictive marker cannot be done retrospectively. Retrospective evaluation can reach the evidence of hypothesis finding studies and/or classify a marker as promising.

Based on the above listed considerations the first prospective randomised clinical trial qualified to evaluate the importance of the functionality of p53 mutations was initiated (see "PANCHO trial" in the example section). These data according to the present invention consistently support that all mutations have functional impact.

The data according to the present invention consistently showed that p53 sequencing completely demarcated the group of non responders supporting that "all mutations have functional impact" teaching.

A specifically preferred method for determining the p53 status of a tumour patient which is characterised in by the following steps:
- providing a sample of body fluid or a tissue sample of the tumour patient containing tumour cells or cell-free tumour DNA; said tumour cells or cell-free tumour DNA containing DNA molecules;
- detecting whether the p53 gene is present in native form on said DNA molecules in said tumour cells or cell-free tumour DNA or whether the p53 gene on said DNA molecules in said tumour cells or cell-free tumour DNA has one or more mutations; said detecting being carried out by:
   - performing on the nucleic acid molecules from said tumour cells or cell-free tumour DNA a quality-controlled, triplicate multiplex polymerase chain reaction (PCR) covering at least exon 2 to exon 11 of the p53 gene of the EMBL sequence U94788 (Seq.ID No. 1), preferably the region from bp 11619 to bp 18741, especially the region from bp 11689 to bp 18598, thereby generating multiplex PCR amplification products;
   - determining the sequence of said triplicate multiplex PCR amplification products by using forward and reverse primers for sequencing (i.e. sequencing each of the three PCR products with forward and reverse primer) thereby generating the sequence of the p53 gene in this region of said tumour cells or cell-free tumour DNA; and
   - comparing the generated sequence with a native p53 gene sequence to detect whether there is at least one mutation present in said tumour cells or cell-free tumour DNA; and
   - determining the p53 status of said tumour patient as mutated or native, depending on whether at least one mutation was detected in the nucleic acids of said tumour cells or cell-free tumour DNA.

The method allows a reliable answer to the question whether the tumour cells or cell-free tumour DNA tested carry a mutation in their p53 gene or not. This is specifically advantageous on the decision for the optimal tumour treatment, especially in view of the qualitative interaction with respect to p53 (see below). With this method, the p53 gene is sufficiently covered so that no false negative or false positive result (which would cause wrong decisions for treatment of the tumour patient) is practically possible. The method is adapted to the needs of practical diagnosis and is suitable for large number testing performed in clinical trials and also in everyday clinical practice.

The present method is based on the reliable determination of the genetic p53 status of a given tumour cell of a tumour patient. This requires the provision of a sample of tumour cells of this patient. Preferably, for defining the p53 status of a patient's tumour a sample of body fluid or a tissue sample of the patient is used which is a blood sample or a tumour biopsy sample (containing histologically verified tumour cells). The present invention provides a reliable determination of the genetic p53 status of a given tumour cell of a tumour patient which requires the provision of a sample of tumour cells of this patient and subjecting this tumour sample to the method according to the present invention, i.e. finding whether a p53 mutation is present in the tumour cell DNA or not. Such a sample can be a tissue specimen, e.g. a tumour biopsy or a suspension of tumour cells harvested by any method, or a sample of a body fluid from such a patient, such as blood (or a blood derived sample, such as serum or plasma), cerebrospinal fluid, lymph, ascitic fluid, or any other body-derived liquid containing tumour cells. The "tumour status" of such cells has to be verified first either by histological or biochemical (immunological) or genetic verification or other means of verification.

The term "quality controlled" has to be understood in that the performance of the PCR is controlled during the reaction. This means that at least one negative control is provided and that the PCR products are analysed (preferably by electrophoretic methods, especially gel electrophoresis). The negative control is preferably a PCR set up with water instead of the DNA (of the sample); of course, also other negative controls can be foreseen, e.g. DNA which should not be polymerised in the PCR can be used as negative control. The negative control serves as a quality control for the exactness of the PCR as well as whether contaminations are present in the stock solutions for the chemicals or in the instruments used; the analysis of the PCR products (especially with respect to their size e.g. by gel electrophoresis) also serves for identifying contaminations or artefacts in the PCR which can interfere with the sequencing step.

The term "quality control" according to the present invention (preferably) also includes that the content of tumour cells histologically verified, the coverage of the p53 gene (amplification of exon 2-11 + intron regions), the triplicate PCR and sequencing; the forward and reverse sequencing, the additional visual inspection, especially by experienced personnel, etc..

The multiplex format allows a cost-effective performance of the method without being too time or workload consuming. Generally, a multiplex-PCR consists of multiple primer sets within a single PCR mixture to produce amplicons of varying sizes that are specific to different DNA sequences within the p53 gene. By targeting multiple genes at once, additional information can be gained from a single test run that otherwise would require several times the reagents and more time to perform.

However, the problem of applying multiplex PCR set-ups in clinical practice is often a lack of reliability and a lack of standardisation ability. Therefore, the method according to the present invention uses a quality-controlled multiplex PCR format which includes a triplicate performance of each PCR test. "Triplicate" means that routinely at least three PCR tests are performed for each set-up, i.e. "triplicate" includes not only "three times" but also "four times", "five times", "ten times" or even more. A person skilled in the art understands that the triplicate multiplex set up according to the present invention sets a new quality standard for p53 testing and that performance and reliability can still be further enhanced by even more parallel set ups; however, the number of set ups has to be weighed with cost and performance considerations. For the method according to the present invention, triplicate performance has proven to be necessary and sufficient for the reliability needed; triplicate testing has to deliver three identical results. If this is not the case, a 10 time testing will allow either a decision or uncover the reason why the test is inconsistent in this certain probe.

Duplicate testing has turned out to be not reliable enough for a standard medical testing method used in clinical practise. On the other hand, e.g. quadruplicate or quintuplicate testing can be even more reliable but such strategy adds costs and effort.
For prevention of false negative results DNA has to be taken separately for each of the triplicate PCR.

For identification of false positive results, negative controls can be foreseen, e.g. by a PCR set up with the same reagents as the samples, but without DNA. If a negative control shows a PCR product, the whole set up must be repeated with new aliquots of reagents and after a (UV-) sterilisation of the work bench.

Annealing temperatures for each of the primer sets must be optimised to work correctly within a single reaction, and amplicon sizes, i.e., their base pair length, should be different enough to form distinct bands when visualised by gel electrophoresis. The amplified nucleic acid must also be suitable for sequencing, e.g. by automated DNA sequencing machines and also other sequencing methods.

The coverage of the p53 gene according to the present invention was carefully chosen to prevent the miss of relevant mutations. Therefore, the method according to the present invention covers at least exon 2 to exon 11 of the p53 gene of the EMBL sequence U94788 (Seq.ID No. 1) thereby also including introns 2 to 10, preferably at least 30 bp adjacent to the respective exon, in order to check all portions of the gene where mutations can eventually be present and relevant for p53 function. Prior art methods have often only observed more specific parts of the p53 gene. Unknown or infrequent mutations in other regions have been missed by such practice. This is excluded by the method according to the present invention. The region including exon 2 to exon 11 (preferably the region from bp 11619 to bp 18741 (covering all amplicons used in the most preferred embodiment), especially from bp 11689 to bp 18598) of the p53 gene is specifically suitable for the method according to the present invention allowing a robust and comprehensive testing of all relevant portions of the p53 gene. A preferred embodiment of the present invention is characterised in that primers are used for amplification of the p53 gene which also include regions (in the resulting amplified molecule) which are at least 10, preferably at least 20, especially at least 30 bp, adjacent to the respective exon. It is, of course also possible to include regions which are at least 50, at least 80, or at least 100 bp (or even more), adjacent to the respective exon. Such primers have significant advantages to primers (such as the IARC primers) which do not allow characterisation of all parts of the exon after forward **and** reverse sequencing. Moreover, with the preferred primers according to the present invention, intron regions are included in which splice site mutations can occur.

A preferred set of primers includes the primers according to SEQ.ID.Nos. 2 to 24.

With the multiplex PCR according to the present invention multiplex PCR amplification products are generated. The sequence of these amplification products ("amplicons") is determined by DNA sequencing. The most convenient and appropriate method for sequencing is automated DNA sequencing. Sequencing according to the present invention is performed by using forward and reverse primers for sequencing for each of the three (or more) multiplex-PCR products. This is also a quality feature and prevents false results, because some mutations can be overlooked if sequencing was performed in the forward or in the reverse only. The result of the sequencing step is the determination of the exact sequence of the p53 gene in the region of said tumour cells which has been amplified by the multiplex PCR.

The comparison of the generated sequence with a native p53 gene sequence (which definitely does not have mutations in the p53 gene) finally allows to come to the result of the present test, namely the identification of one or more specific mutations in the p53 gene or the verification that the cancer cell tested does not have a mutation in the p53 gene. This comparison can be done automatically by various computer programs; however it is an additional and preferred quality control step to inspect the sequences visually, e.g. by experienced sequencing experts, in order to interpret suboptimal or inconclusive data and/or to make the decision for resequencing. Usually, after finishing the sequence run the raw data (e.g. the fluorescence signals) can be stored, analysed and transferred in a sequence format. Depending on the program used, the raw data (e.g. SeqScape) or the analysed sequence (e.g. Autoassembler, SeqScape) are used for the comparison. However, the method according to the present invention is not dependent on a specific sequencing platform and can be applied in any sequencing method (ABI, Beckmann, etc.), as long as a comparison of a multitude of (at least more than one) sequence runs can be performed on different samples and compared with each other.

The p53 status of a tumour patient will be determined as mutated if at least one mutation was detected in the nucleic acids of said tumour cells by the method according to the present invention. If no mutation was detected, the p53 status of this patient will be determined to be native. Overall therapy depends on the primary tumour, the primary tumour is therefore the basis for the assay according to the present invention. If a tumour shows synchronic metastasis, p53 status of the metastases is unchanged. If, nevertheless, different mutation status should occur in a patient this could be due to two different primary tumours. In such exceptional cases, the two possible optimal therapy regimes have to be fine-tuned to each other (e.g. local irradiation for the non-mutated tumour and pathway 2 therapy for mutated tumours; or sequential administration of chemotherapies).

According to a preferred embodiment, the multiplex PCR in the method according to the present invention is performed with primers having a melting temperature of 58°C to 72°C, preferably of 60°C to 70°C, especially of 65°C to 68°C. This temperature/primer combination is especially suitable for standard testing in clinical practice. Optimum melting temperatures can be determined for a given primer set by a person skilled in the art, mainly based on the primary sequence to be analysed and on the salt concentration of the buffers.

Multiplexing the PCR allows a time and effort saving performance of the method according to the present invention. However, care must be taken that the PCR is not "overloaded" with primers and sample DNA, because this could lead to false negative results (if a given sub-reaction did not properly work) or amplification artefacts (which could produce a high background signal and interference with sequence analysis). Care must be taken to adjust appropriate number of different amplifications in one reaction, the lengths of the amplicons, the number of PCR cycles, etc. Multiplex PCR is performed with at least two different primer pairs (= four primers); such multiplex PCR resulting in at least two independent PCR products. The multiplex PCR according to the present invention is preferably performed with a total (for a given test of a patient's cells) of at least 8 or of at least 10, preferably at least 15, especially at least 20, primer pairs covering different regions of the p53 gene. These primer pairs are then provided in combinations of two primer pairs or more in suitable multiplex set-ups. In order to make the multiplex PCR according to the present invention efficient with respect to time and effort, the totality of the primers in the multiplex PCR is performed with 5 or less independent PCRs, more preferred with 4 or less independent PCRs, especially with 3 or less independent PCRs. Having a number of at least 10, especially at least 20 primer pairs, provision of three independent PCRs has shown to be the most preferred embodiment; only two or even only one PCR reaction for all the primer pairs has drawback with respect to complexity, especially in obtaining the re-suits and compatibility with the sequencing step thereafter.

A primer set has been developed for the present method which provides specifically suitable reliability and performance for determination of the p53 status of a tumour patient. This primer set has been designed for the clinical testing of the present invention and therefore fully serves the needs of the present invention. Therefore, according to a preferred embodiment of the present invention, these primers are used for carrying out the present invention. A preferred embodiment of the method according to the present invention is therefore characterised in that at least one, preferably at least three, especially at least five, primer pair(s) of the primer pairs according to SEQ.ID.Nos. 2 and 4 to 22 is/are used in said triplicate multiplex PCR and/or said sequence determination. With the exception of the primers for exon 4 (SEQ.ID.Nos. 7 and 8), these PCR primers are also used in the most preferred embodiment for sequencing (for exon 4, the sequencing primers SEQ.ID.Nos. 23 and 24 are used).

A specifically preferred embodiment of the invention is characterised in that the primer pairs according to SEQ.ID.Nos. 2 to 24 are used in said triplicate multiplex PCR and/or said sequence determination (again, with the peculiarities concerning exon 4). It is also clear to a person skilled in the art that the primers herein can be slightly amended (e.g. shifting some (e.g. 1, 2, 3, 4, or 5) base pairs 5' or 3' along the p53 sequence) usually without much difference, nevertheless, the primers disclosed herein represent a specifically preferred embodiment.

Preferably, the p53 status determination according to the present invention is performed by running in parallel at least a positive and/or a negative control. Preferred positive controls are a tumour cell or a cell-free DNA with a p53 gene in native form and/or a tumour cell or a cell-free DNA with a mutated p53 gene. Preferably, the nature and details of the p53 mutation is known; also DNA with a p53 gene with more than one mutation can be applied as a positive control. Such positive controls are useful as markers for the appropriate working of the amplifications and/or the detectability of wild type or mutant p53 gene. Preferred negative controls run in parallel to the determination of the p53 status of the tumour patient are DNA free of sequences that are amplified during the triplicate multiplex PCR and/or a DNA free solution. The "DNA free of sequences that are amplified during the triplicate multiplex PCR" is a DNA which, under appropriate working of the PCR reaction does not result in any amplification product with the specific primers applied. Creation of an amplification signal in such negative control implies then either contamination by another DNA or unsuitable PCR conditions (too low stringency; too low polymerase specificity, etc.). It is clear that positive and negative control PCRs have to be carried out identically (stringency, polymerase specificity, etc.) to the sample PCRs. As already stated above, it is preferred to use the same primers for a given triplicate multiplex PCR and for the determination of the sequence of said triplicate multiplex PCR amplification products, i.e. the sequencing primers for a given amplicon are the same as for the PCR. This applies for most of the primers/amplicons (except for exon 4, where the amplicon spans a repetitive sequence which has to be excluded in sequence analysis). Accordingly, at least one, preferably at least three, especially at least five primer pairs for the PCR are also used for the sequencing.

Disclosed is also a kit for performing the method according to the present invention. The kit comprises:
- a PCR primer set, preferably with at least one, more preferred at least three, especially with at least five primer pairs (forward/reverse) of SEQ.ID.Nos. 2 to 24;
- optionally, PCR reagents, including a DNA polymerase, buffer(s), and dNTPs; and
- a sequencing primer set.

This kit can be packaged and provided in a "ready to use" format so that it is applicable in any diagnosis laboratory to determine the p53 status of a tumour patient. PCR reagents, including a DNA polymerase, buffer(s), and dNTPs, can be provided in the kit; however, it is also established practice that such reagents are supplied separately (e.g. ingenetix MSI Panel PCR Kit), so that the PCR kits are commercialised with the primers only. Of course, for performing the method according to the present invention, these reagents have to be present.

In a preferred embodiment, the kit further comprises control reagents, preferably positive control reagents, especially a tumour cell or a cell-free DNA with a p53 gene in native form and/or a tumour cell or a cell-free DNA with a mutated p53 gene, or negative control reagents, DNA free of sequences that are amplified during the triplicate multiplex PCR and/or a DNA free solution.

Preferably, the kit contains the primers with SEQ.ID.Nos. 2 to 24.

Often, the PCR reagents and primers as well as the polymerase are optimised with respect to a given thermocycler. It is therefore practical, if the kit also comprises a thermocycler ready to be used with the other components of the kit. Preferably, the other components of the kit, especially the buffers, PCR primers and the polymerase have been optimised for the given thermocycler.

Preferred kits already contain the primers in prepared multiplex mixtures so that the primers do not have to be added separately to the PCR mix but are already provided in the appropriate multiplex mixture (i.e. in the optimised concentrations).

The invention is further described in the following examples and the drawing figures, yet without being restricted thereto.
Figure 1 shows p53 mutation rate in oesophageal cancer: Percentage of mutated tumours reported by (A) p53 Research, (B) IARC p53 Database, (C) UMD p53 Database;
Figure 2 shows gelelectrophoresis of multiplex PCR products: (A) Exons 5, 2, 8 and 7 are amplified with primermix M1, (B) Exons 6, 3 and 11 with primermix M2, (C) Exons 4, 10 and 9 with primermix M3. Fragment sizes are specified in basepairs (bp);
Figure 3 shows the sequencing data of samples 2234 and 2235 (A) Forward sequencing: Both mutations are visible (hatched and dotted arrow); (B) Reverse sequencing; mutation sample 2234 barely visible, but the peak height is lower than normal as shown in sample 2235 (hatched arrow), mutation sample 2235 visible (dotted arrow); all mutated positions show a lower height of the normal peak compared to the neighbouring peaks and the sequence without a mutation;
Figure 4 shows the primers used in the examples section of the present invention in comparison with the IARC primers;

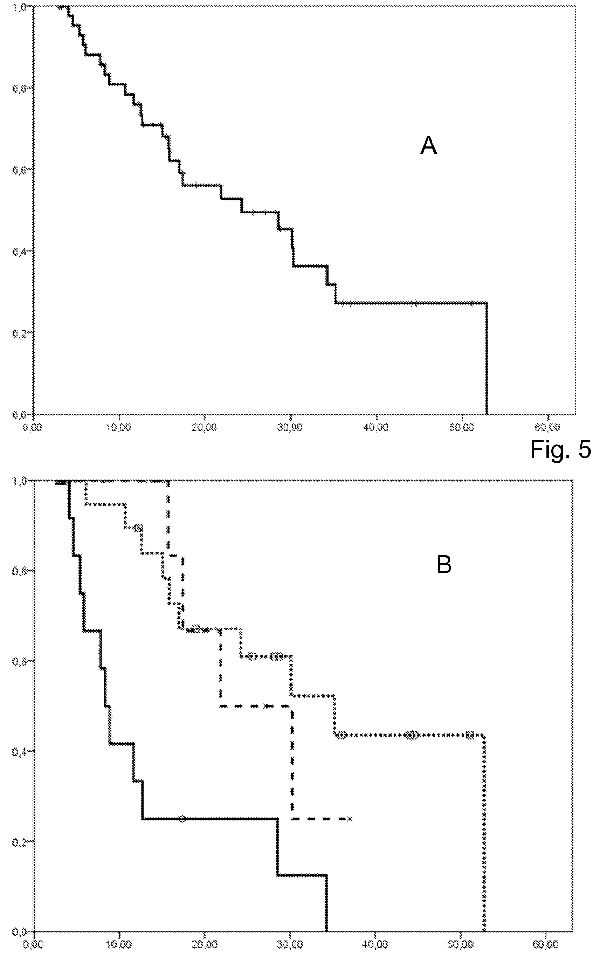

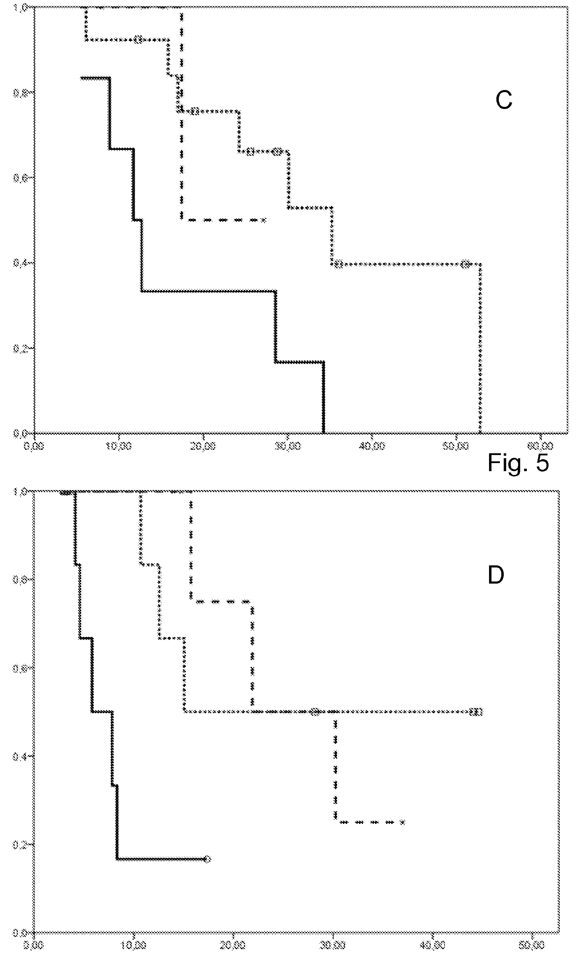

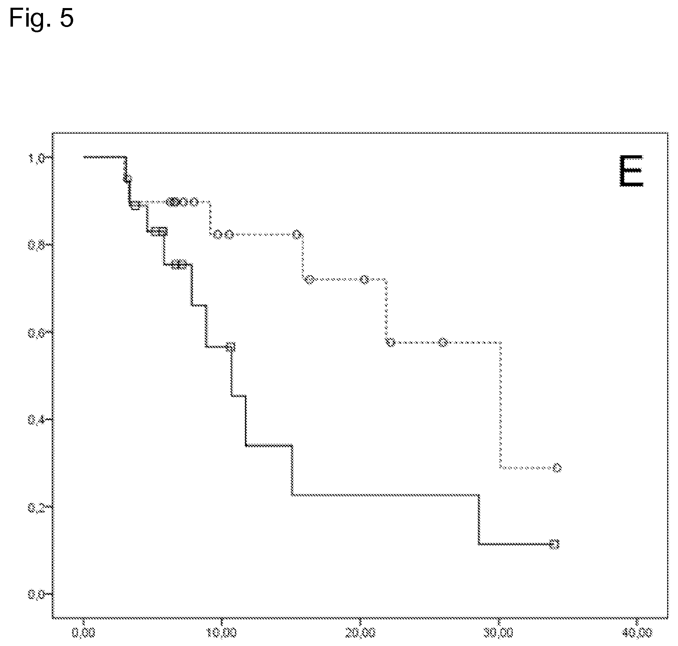

Figure 5 Data from Key Study: Oesophageal cancer pilot study: (A) Overall survival of neoadjuvantly treated oesophageal cancer patients (n=47); treatment was either Cisplatin/5-FU (n=36) or Docetaxel (n=11); mean survival rate was 24.2 months; (B) survival of patients with p53 normal tumours and Cis/5-FU treatment (dotted line; mean survival 34 months); p53 mutated tumours and Cis/5-FU treatment (full line; mean survival 14 months); p53 mutated tumours and Docetaxel treatment (dashed line; mean survival 26 months) ; p<0,001; (C) line shapes are analogous to (B); mean survival of patients with adenocarcinoma (n=24) was 35 (dotted line) vs. 17 (full line) vs. 22 months (dashed line); p=0.024; (D) line shapes are analogous to (B); mean survival of patients with squamous cell carcinoma (n=23) was 26 (dotted line) vs. 8 (full line) vs. 29 months (dashed line); p=0.01; (E) overall survival of patients treated with p53 adjusted therapy (dotted line; mean survival 30 months) and p53 non adjusted therapy (full line; mean survival 15 months); p=0.042;

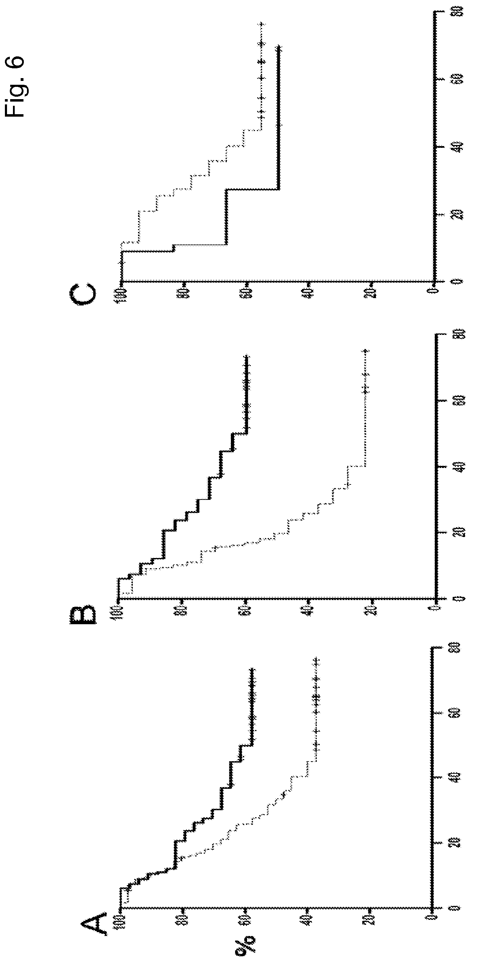

Figure 6 shows data from Key Study: CRCLM (colorectal cancer liver metastases) : full line: p53 normal; dotted line : p53 mutant; (A) survival of patients with CRCLM (n=76) with normal p53 (full line) and mutant p53 (dotted line); (B) subset of patients treated with preoperative chemotherapy 5-FU/Oxaliplatin (n=51) with normal p53 (full line) and mutant p53 (dotted line); p=0.025; Cox-model was used to calculate Hazard ratio 3.24 (95%CI 1.5-7.0); p=0.045; adjustment to known prognostic parameters (age, sex, T-stage, N-stage, grading, synchronous/metachronous tumours), results in a hazard ratio of 5.491 (95%CI 2.28-13.24); p=0.0042; (C) subset of patients without preoperative Chemotherapy (no chemotherapy); normal p53 (full line) is not related to improved survival; p=0.543; patients receiving preoperative chemotherapy show better survival than those receiving no chemotherapy in case they have a normal p53 gene (full lines (B) and (C)); patients receiving preoperative chemotherapy do worse than patients receiving no chemotherapy in case they have a p53 mutation (dotted lines (B) and (C)); patients with p53 mutated tumours receiving 5-FU/oxaliplatin chemotherapy have a 5.49 fold risk to die (p=0.042);
Figure 7 shows marker by treatment interaction design to test a predictive factor question: Sargent et al., J. Clin. Oncol. 23 (2005), 2020-2027;

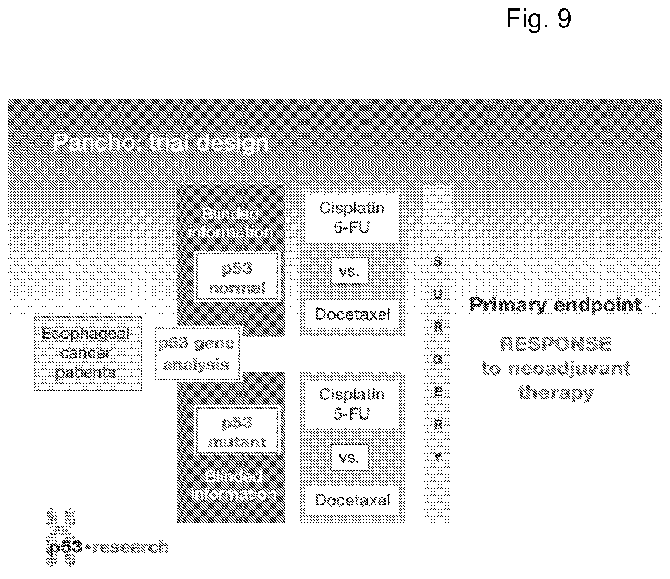

Figure 8 shows cumulatively reported number of mutations in the years 1985 to 2008: full line - all mutations, line with squares - all new mutations, line with triangles - new missense mutations;
Figure 9 shows PANCHO: the trial design: Eligible for the PANCHO trial are operable oesophageal cancer patients >T1 stage. P53 gene analysis is performed as marker test. Patients are stratified for their histological type (adeno-, squamous cell carcinoma); marker negative patients (p53 normal) are randomised to receive either Cispaltin/5-FU or Docetaxel preoperatively; marker positive patients are also randomised to receive either Cisplatin/5-FU or Docetaxel; after three cycles of preoperative chemotherapy all patients are referred to surgery; response to neoadjuvant therapy is defined as primary endpoint and is assessed comparing the diagnostic tumour stage with the pathological tumour stage.

### EXAMPLES:

### I.: Clinical Evaluation:

Reports from exploratory studies regularly suggest potentially useful candidate markers to optimise and individualise cancer therapy. However, few markers are currently developed to the point of allowing reliable use in clinical practice. The lack of a disciplined approach will slow the introduction of markers into clinical use, or alternatively, markers may be introduced without sufficient scientific evidence of benefit. Once the marker meets the criterion of "promising", additional data must be gathered before initiating confirmatory studies to test its clinical utility. These data include the specificity of the marker to the cancer of interest (as opposed to normal tissues, other disease states, or other cancers), an estimate of the marker prevalence in the target population, confidence in the method of measurement, including definition of any cut points, and demonstration that the measurement can be reliably performed on the specimens that are available (Sargent et al., J. Clin. Oncol.23 (2005), 2020-2027).

Considering these development milestones a number of studies were performed to evaluate the clinical utility of p53 stepwise. While performing these studies (Examples 1-3) the qualitative interaction was detected and the two pathway model was developed. Both hypotheses are now finally tested in the first prospective randomised clinical phase III trial (pancho trial) appropriately designed to test for qualitative interaction.

### 1. The CRCLM (Colorectal cancer liver metastases) Study

Goal:
- Test for an independent association of the marker p53 and chemotherapy response
- get information about the prognostic properties of the marker by including an untreated control group
- get evidence for the qualitative interaction

Data on 76 patients with colorectal cancer liver metastases (CRCLM) were prospectively collected at a single institution between 2001 and 2003. Patients considered to be technically operable were included. Fifty-one patients received preoperative therapy with Oxaliplatin plus 5-FU and twenty-five were treated with surgery only. The groups did not differ in age, chronicity of CRCLM, staging and grading of the primary colorectal cancer. Treatment decision was based on the preference of the surgeon or the patient.

The p53 gene was assessed in all tumours through complete direct gene sequencing (exons 2-11 including splice sites).

In Figure 6A survival rates for the whole patient cohort are shown (the graph includes all patients with and without preoperative chemotherapy and separates them for harbouring p53 mutant and p53 normal tumours). In this graph a normal p53 seems to be beneficial. However, subgroup evaluation shows: Improved survival did occur in patients with p53 normal tumours but exclusively in the group receiving preoperative chemotherapy (p=0,025) (Figure 6B). The chemotherapy used was 5FU/Oxaliplatin. Both drugs belong to pathway 1 and need a normal p53 for induction of apoptosis.

In contrast, in the preoperatively untreated control group (Figure 6C), a normal p53 status in the tumour was not related to improved survival (p=0.543). Quite contrary, the effect seems to be inverse.

Comparing only the dotted lines of both subsets (Figures 6B and 6C), representing the patients with a p53 mutant tumour, patients with preoperative chemotherapy (Figure 6B) did much worse. These patients received drugs (5FU/Oxaliplatin) which belong to pathway 1. The graph shows that the pathway 1 chemotherapy was ineffective in patients with p53 mutated tumours. The graph additionally shows that pathway 1 chemotherapy harmed patients with p53 mutated tumours because patients with p53 mutated tumours and without preoperative chemotherapy did better.

Comparing the full lines of the two subsets with and without preoperative chemotherapy (representing the patients with p53 normal tumours), survival benefit was related to preoperative chemotherapy.

In summary, the used chemotherapeutic drugs belonging to pathway 1 interact positively with a normal p53 gene and negatively with a mutant p53 gene.

In other words, depending on the genotype of the marker (mutant or normal) the effect of therapy changes direction, which demonstrates the presence of a qualitative interaction. The degree of interaction is even stronger (p=0.0042) when the known prognostic parameters are considered in the statistical calculation (multivariate analysis). The p53 genotype shows a significant (qualitative) interaction with survival (response to therapy) in the chemotherapy treated subset only.

The p53 genotype of the tumour was only related to survival in patients receiving chemotherapy demonstrating that the marker p53 interacts with chemotherapy. This qualifies p53 as a predictive marker. P53 did not influence survival in the preoperatively untreated patients and therefore p53 does not qualify as a prognostic marker.

In summary, these results show that:
- p53 exclusively interacts with chemotherapy.
- p53 is not a prognostic marker but a marker predicting response to chemotherapy.
- p53 can easily be misinterpreted as a prognostic marker when the strong interaction with chemotherapy is not considered (see Fig 6A). Today almost all cancer patients are treated (pre or postoperatively) and most of the frequently used chemotherapies interact negatively with a mutant p53. Therefore in meta-analyses a mutant p53 status may appear as a bad prognostic marker.
- Any survival benefit attributed to p53 is based on its interaction with therapy.

### 2. The Oesophageal cancer PILOT STUDY

Goal:
- Validation trial for the relationship between marker genotype and outcome (finished 2007)
- test p53 adapted preoperative therapy (treatment considering the two pathway model) for the first time prospectively
- assess the true incidence of p53 mutations in oesophageal cancer.

Background: Treatment of oesophageal cancer remains unsatisfactory. Cure rates are disappointingly low. The median survival time ranges around 17 months with a 3 ys survival rate of only 16%. Neither pre nor postoperative radio/chemotherapy in any combination proved to substantially improve this situation.

Only a small subgroup of patients who experience major response to preoperative therapy consistently shows a significantly increased survival. Using standard platinum-based regimen, yet about 15 % of patients can achieve pathological complete remission which translates in reported 3-year survival rates of 64 % in this group. Factors identifying this subgroup of responders and selecting optimal drugs for non-responders could therefore dramatically enhance treatment efficacy.

Methods: In order to test the hypothesis that the p53 genotype is predictive for chemotherapy response, a prospective study was conducted. Thirty-eight patients with potentially re-sectable esophageal cancer were evaluated for the relation between p53 genotype and response to two different neoadjuvant treatments. P53 gene mutations were assessed by complete direct sequencing of DNA extracted from diagnostic biopsies. Response to neoadjuvant chemotherapy was assessed pathohistologically in the surgical specimen.

Results: 23 squamous cell carcinoma and 24 adenocarcinoma were included. 39 patients received standard therapy with CIS/5FU (Cisplatin 80mg/m2 d1 5-FU 1000mg/m2 d 1-5, q21,2 cycles). Eight patients received Docetaxel (75mg/m2, q21,2 cycles).

Presence of a p53 mutation was significantly associated with decreased survival in the group receiving 5FU/CIS and an increased survival in the group receiving Docetaxel (Fig 5B). Patients with a normal p53 gene experience a significant survival benefit after 5FU/CIS therapy.

**Table 3**

| | CISPLATIN / 5-FU | | | | | DOCETAXEL | | | |
|---|---|---|---|---|---|---|---|---|---|
| | | | | | | | | | |
| | p53 NORMAL | | P53 MUTANT | **P VALUE** | | P53 NORMAL | | P53 MUTANT | **P VALUE** |
| RESPONSE: CR, PR | **12** | | **0** | | | **0** | | **6** | |
| FAILURE: 3D, PD | **2** | | **16** | <0.001 | | **2** | | **0** | 0.002 |

The overall response to p53 adapted neoadjuvant therapy was 94%. p53 adapted treatment was associated with a significant survival advantage (p=0,042) after a median follow up of 15.4 months (Fig 5E). There was no difference according to the different histological subtypes concerning the p53 interaction (Fig 5C, 5D).

Conclusion: These results are in concordance with our interaction and pathway model: As CIS/5FU belongs to pathway 1 and worked well in patients with a normal p53 gene in the tumour. Docetaxel belongs to pathway 2 and worked well in patients with p53 mutant tumours.

As a consequence, a prospective randomised trial - the PANCHO trial- was initiated to finally prove the interaction between the predictive marker p53 and response to CIS/5-FU and Docetaxel, respectively.

### 3. The PANCHO Trial

Goal:
- Provide clinical evidence for the qualitative interaction and the two pathway model.
- prove the clinical utility of p53 for the first time in a prospective randomised, clinical phase III trial.
- use the Marker by treatment interaction design proposed by Sargent and test for interaction between p53 and response to therapy for the first time in the context of a phase III trial

PANCHO = "p53 adapted neoadjuvant chemotherapy for operable oesophageal cancer" EudraCT 2006 006647-31, NCT00525200) is an ongoing clinical, predictive marker trial conducted by the p53 research group (started 2007, scheduled to be finished 2011).

The trial was designed to provide clinical evidence for the two pathway model and the qualitative interaction of p53 and anticancer therapy.

There is no single marker known for which a direct qualitative interaction with cancer therapy has ever been shown in a clinical trial. Additionally, based on the two pathway model of p53 interacting with cancer therapy, this interaction is two sided.

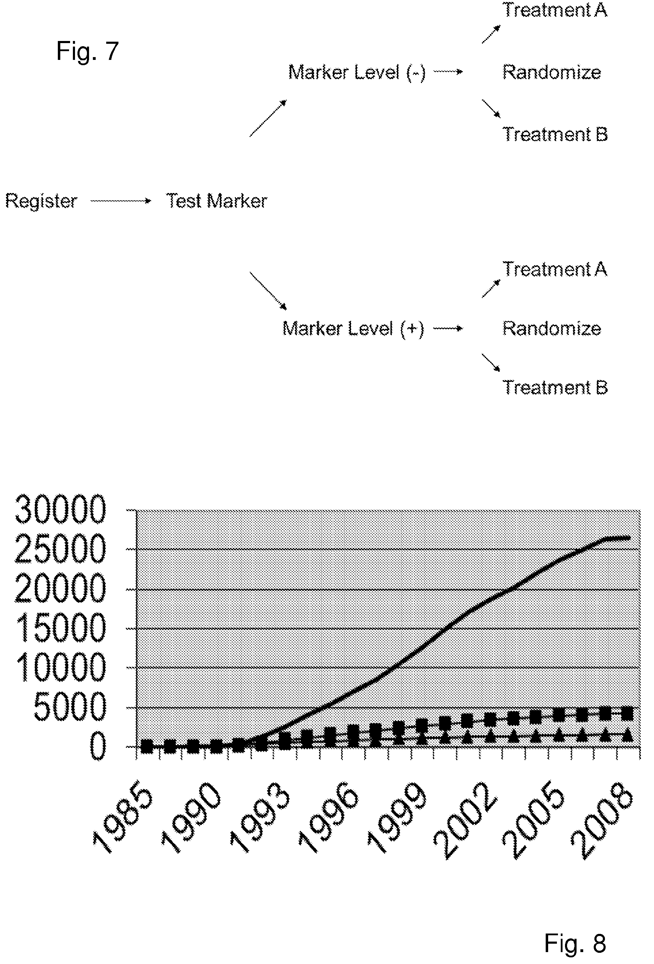

The design of the pancho trial - marker by treatment interaction design (Sargent et al., J. Clin. Oncol. 23 (2005), 2020-2027) - was proposed as the statistically adequate design to test a possible interaction between a marker and response (Fig. 7 and 9).

Until the present invention this design has never been used in a clinical trial because still no marker has been identified to meet the prerequisites: availability of a potential effective therapy for each of the two marker expressions (mutant or wild type) which generates a huge difference in response.

The qualitative two sided interaction model according to the present invention will change the standards of cancer therapy, reducing toxicity while improving efficacy.

### II. Determination of the p53 status:

### 1. Background

The p53 gene is located on the short arm of chromosome 17 in the region 17p13.1. The genomic region spans approximately 22 kb where the coding sequence is arranged in 11 exons. Start of the translation for the 2.2 kb mRNA is in exon 2 with the first nucleotide at position 11717 and the last nucleotide at position 18680 (exon 11) of the sequence with the accession number U94788 (Seq.ID.No.1). Detailed information on the size and location of exonic and intronic regions according to the published sequence is given in Table 4.

**Table 4: p53 - exons and introns**

| | number | size (bp) | nt start¹ | nt end¹ |
|---|---|---|---|---|
| exon | 2 | 102 | 11689 | 11790 |
| exon | 3 | 22 | 11906 | 11927 |
| exon | 4 | 279 | 12021 | 12299 |
| exon | 5 | 184 | 13055 | 13238 |
| exon | 6 | 113 | 13020 | 13432 |
| exon | 7 | 110 | 14000 | 14109 |
| exon | 8 | 137 | 14452 | 14588 |
| exon | 9 | 74 | 14684 | 14754 |
| exon | 10 | 107 | 17572 | 17678 |
| exon | 11 | 82 | 18599 | 18680 |
| intron | 2 | 115 | 11791 | 11905 |
| intron | 3 | 93 | 11928 | 12020 |
| intron | 4 | 755 | 12300 | 13054 |
| intron | 5 | 81 | 13239 | 13319 |
| intron | 6 | 567 | 13433 | 13999 |
| intron | 7 | 342 | 14110 | 14451 |
| intron | 8 | 92 | 14589 | 14680 |
| intron | 9 | 2817 | 14755 | 17571 |
| intron | 10 | 920 | 17679 | 18598 |

| | | | | |
|---|---|---|---|---|
| Nucleotide position according to sequence U94788 | | | | |

### 2. Primer design for multiplex PCR

Primers were designed with the Primer3 software package (Steve Rozen and Helen J. Skaletsky (2000) Primer3 on the WWW for general users and for biologist programmers. In: Krawetz S, Misener S (eds) Bioinformatics Methods and Protocols: Methods in Molecular Biology. Humana Press, Totowa, NJ, pp 365-386). Melting temperature of primers was set to range from 65 to 68 °C and there should preferably be a distance of at least 30 bp between primers and exon sequence. The melting temperature depends on the DNA sequence of the primer region and has to be lower than 72°C, which is the optimal temperature for most polymerases used for PCR amplification. At the same time melting temperature has to be as high as possible to prevent amplification of products outside of the region of interest where a primer has bound only partially. A uniform annealing temperature of all primer-pairs used for p53 amplification allows simultaneous amplification of several fragments in a single reaction. The distance between primer position and exon sequence is essential to guarantee analysis of the whole coding sequence including splice sites at the intron-exon-borders.

Furthermore to allow sequence analysis of DNA fragments amplified simultaneously in one reaction primer-binding sites must not lead to overlapping amplicons.

Detailed information on the amplicons and primer sequences is given in Table 5.

**Table 5: p53 - amplicons and primers for PCR**

| | number | size (bp) | nt start1 | nt end1 | sequence |
|---|---|---|---|---|---|
| amplicon | 2 | 250 (266) | 11619 | 11868 | |
| amplicon | 3 | 234 | 11833 | 12066 | |
| amplicon | 4 | 418 | 11937 | 12354 | |
| amplicon | 5 | 300 | 12991 | 13290 | |
| amplicon | 6 | 251 | 13245 | 13495 | |
| amplicon | 7 | 239 | 13926 | 14164 | |
| amplicon | 8 | 250 | 14391 | 14640 | |
| amplicon | 9 | 243 | 14608 | 14850 | |
| amplicon | 10 | 286 | 17464 | 17749 | |
| amplicon | 11 | 216 | 18526 | 18741 | |
| primer f | 2 | 21 | 11619 | 11638 | (gatcgatcgatcgatc) |
| ttctctgcaggcccaggtga (Seq.ID.No.2/3) | | | | | |
| primer r 2 (Seq.ID.No.4) | | 21 | 11848 | 11868 | tcgcttcccacaggtctctgc |
| primer f 3 (Seq.ID.No.5) | | 20 | 11833 | 11852 | aaccccagccccctagcaga |
| primer r 3 (Seq.ID.No.6) | | 20 | 12047 | 12066 | ccggggacagcatcaaatca |
| primer f 4 (Seq.ID.No.7) | | 19 | 11937 | 11955 | agggttgggctggggacct |
| primer r 4 (Seq.ID.No.8) | | 21 | 12334 | 12354 | gggatacggccaggcattga |
| primer f 5 (Seq.ID.No.9) | | 29 | 12991 | 13017 | ccagttgctttatctgttcacttgtgc |
| primer r 5 (Seq.ID.No.10) | | 18 | 13273 | 13290 | ctggggaccctgggcaac |
| primer f 6 (Seq.ID.No.11) | | 20 | 13245 | 13264 | agctggggctggagagacga |
| primer r 6 (Seq.ID.No.12) | | 19 | 13477 | 13495 | ccggagggccactgacaac |
| primer f 7 (Seq.ID.No.13) | | 20 | 13926 | 13945 | aaaaggcctcccctgcttgc |
| primer r 7 (Seq.ID.No.14) | | 19 | 14146 | 14164 | aagcagaggctggggcaca |
| primer f 8 (Seq.ID.No.15) | | 24 | 14391 | 14414 | tgggacaggtaggacctgatttcc |
| primer r 8 (Seq.ID.No.16) | | 23 | 14618 | 14640 | ggcataactgcacccttggtctc |
| primer f 9 (Seq.ID.No.17) | 20 | 14608 | | | 14627 agcggtggaggagaccaagg |
| primer r 9 (Seq.ID.No.18) | 22 | 14829 | | | 14850 tgccccaattgcaggtaaaaca |
| primer f 10 (Seq.ID.No.19) | 23 | 17464 | | | 17486 tcgatgttgcttttgatccgtca |
| primer r 10 (Seq.ID.No.20) | 25 | 17725 | | | 17749 aatggaatcctatggctttccaacc |
| primer f 11 (Seq.ID.No.21) | 20 | 18526 | | | 18545 ggtcagggaaaaggggcaca |
| primer r 11 (Seq.ID.No.22) | 20 | 18722 | | | 18741 tggcaggggagggagagatg |
| primer seq f 4 (Seq.ID.No.23) | 19 | 11992 | | | 12010 ctctgactgctcttttcac |
| primer seq r 4 (Seq.ID.No.24) | 20 | 12321 | | | 12340 cattgaagtctcatggaagc |

| | | | | | |
|---|---|---|---|---|---|
| Nucleotide position according to sequence U94788; f forward, r reverse; primer f 2 contains a 4 x gatc elongation; this is a preferred embodiment to provide a better distinguishability between exons 2 and 8 in mix 1 without influence on primer binding an reaction conditions. | | | | | |

### 3. Multiplex PCR amplification

With the primers listed in Table 6 all coding exons of the p53 gene can be amplified in 3 PCR reactions followed by individual sequence analyses. Because of a repetitive sequence in intron 3 which had to be included in the amplicon due to proximity to the start of the exon, another forward primer had to be chosen for sequence analysis of exon 4. The reverse primer for exon 4 sequencing also differs from that used for PCR amplification, as it gave improved results - which were shown in stronger signals and less background. All other exons can be sequenced with the same primers used for PCR amplification.

The forward primer of exon 2 was elongated at the 5-prime end by a non-complementary fragment of 4 GATC-series to give a distinguishable band in polymerase gel electrophoresis. This allows a quality test for each amplification reaction.

All PCR-amplifications are optimised to be performed in a Biometra Thermocycler T1 or T-gradient (Biometra, Göttingen, Germany).

### 3.1 p53 amplification mix M1

In the mix M1 exons 2, 5, 7 and 8 are amplified simultaneously in one reaction. Primers are stored in stock solutions of 100 µM. A working solution containing the respective concentration ratio is prepared from 10 µM solutions; 2.6 µl of the working solution are added to each amplification reaction.

**Table 6: Composition of the PCR-reaction for mix M1**

| amount¹ | substance | supplier²/cat-number |
|---|---|---|
| to a total volume | pure water | Merck/116434 |
| of 50 µl | | |
| 6 µl | Buffer I | Applied Biosystems/N8080244 |
| 250 µM | dNTP | Applied Biosystems |
| 2,5 U | Ampli Taq | Applied Biosystems/N8080244 |
| | Gold Polymerase | |
| 80 µM | primer 2f | Sigma-Genosys |
| 80 µM | primer 2r | Sigma-Genosys |
| 60 µM | primer 5f | Sigma-Genosys |
| 60 µM | primer 5r | Sigma-Genosys |
| 60 µM | primer 7f | Sigma-Genosys |
| 60 µM | primer 7r | Sigma-Genosys |
| 60 µM | primer 8f | Sigma-Genosys |
| 60 µM | primer 8r | Sigma-Genosys |
| 4 µl | sample DNA | |

| | | |
|---|---|---|
| ¹ Reaction volume is 50 µl ² Applied Biosystems, Foster City, CA; Merck, Darmstadt, Germany; Sigma-Aldrich, Vienna, Austria. | | |

**Table 7: Cycling protocol for mix 1**

| step | temperature (°C) | time (s) | ramp (°C/s) | no of cycles¹ |
|---|---|---|---|---|
| 1 | 95 | 600 | 5 | |
| 2 | 95 | 40 | 5 | |
| 3 | 64 | 40 | 5 | 47 |
| 4 | 75 | 60 | 3 | |
| 5 | 72 | 600 | 5 | |
| 6 | 15 | hold | 5 | |

| | | | | |
|---|---|---|---|---|
| ¹ One cycle include steps 2 to 4 which are repeated the respective times | | | | |

### 3.2 p53 amplification mix M2

In the mix M2 exons 3, 6 and 11 are amplified simultaneously in one reaction. Primers are stored in stock solutions of 100 µM. A working solution containing the respective concentration ratio is prepared from 10 µM solutions; 2.4 µl of the working solution are added to each amplification reaction.

**Table 8: Composition of the PCR-reaction for mix M2**

| amount¹ | substance | supplier²/cat-number |
|---|---|---|
| to a total | pure water | Merck/116434 |
| volume of 30 µl | | |
| 15 µl | QIAGEN® Multiplex | Qiagen/206145 |
| | PCR Kit | |
| 40 µM | primer 3f | Sigma-Genosys |
| 40 µM | primer 3r | Sigma-Genosys |
| 80 µM | primer 6f | Sigma-Genosys |
| 80 µM | primer 6r | Sigma-Genosys |
| 120 µM | primer 11f | Sigma-Genosys |
| 120 µM | primer 11r | Sigma-Genosys |
| 4 µl | sample DNA | |

| | | |
|---|---|---|
| ¹ Reaction volume is 30 µl ² Merck, Darmstadt, Germany; Qiagen, Hilden, Germany; Sigma-Aldrich, Vienna, Austria. | | |

**Table 9: Cycling protocol for mix 2.**

| | temperature (°C) | time (s) | ramp (°C/s) | no of cycles¹ |
|---|---|---|---|---|
| 1 | 95 | 600 | 5 | |
| 2 | 95 | 40 | 5 | |
| 3 | 64 | 90 | 5 | 47 |
| 4 | 76 | 40 | 3 | |
| 5 | 72 | 600 | 5 | |
| 6 | 15 | hold | 5 | |

| | | | | |
|---|---|---|---|---|
| ¹ One cycle include steps 2 to 4 which are repeated the respective times | | | | |

### 3.3 p53 amplification mix M3

In the mix M3 exons 4, 9 and 10 are amplified simultaneously in one reaction. Primers are stored in stock solutions of 100 µM. A working solution containing the respective concentration ratio is prepared from 10 µM solutions; 2.3 µl of the working solution are added to each amplification reaction.

**Table 10: Composition of the PCR-reaction for mix M3.**

| amount¹ | substance | supplier²/cat-number |
|---|---|---|
| to a total | pure water | Merck 116434 |
| volume of 50 µl | | |
| 25 µl | QIAGEN® Multiplex | Qiagen/206145 |
| | PCR Kit | |
| 100 µM | primer 4f | Sigma-Genosys |
| 100 µM | primer 4r | Sigma-Genosys |
| 30 µM | primer 9f | Sigma-Genosys |
| 30 µM | primer 9r | Sigma-Genosys |
| 100 µM | primer 10f | Sigma-Genosys |
| 100 µM | primer 10r | Sigma-Genosys |
| 4 µl | sample DNA | |

| | | |
|---|---|---|
| ¹ Reaction volume is 50 µl ² Merck, Darmstadt, Germany; Qiagen, Hilden, Germany; Sigma-Aldrich, Vienna, Austria. | | |

**Table 11: Cycling protocol for mix 3.**

| | temperature (°C) | time (s) | ramp (°C/s) | no of cycles¹ |
|---|---|---|---|---|
| 1 | 95 | 600 | 5 | |
| 2 | 95 | 40 | 5 | |
| 3 | 65-1/cycle² | 90 | 5 | 5 |
| 4 | 75 | 60 | 3 | |
| 5 | 95 | 40 | 5 | |
| 6 | 60 | 90 | 5 | 42 |
| 7 | 75 | 60 | 3 | |
| 8 | 72 | 600 | 5 | |
| 9 | 15 | hold | 5 | |

| | | | | |
|---|---|---|---|---|
| ¹ One cycle include steps 2 to 4 and afterwards steps 5 to 7 which are repeated the respective times ² Annealing temperature of the first cycle is 65 °C, in each of the following cycles the temperature is lowered for 1° | | | | |

### 4. PCR amplification quality control

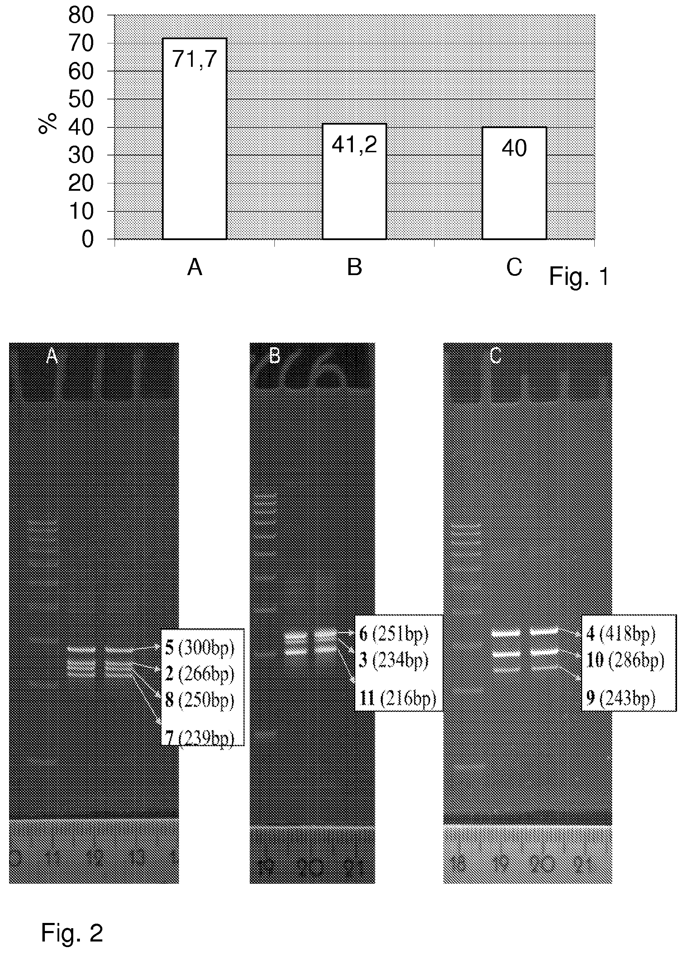

For quality and quantity assessment PCR products are analyzed on precast 5 % acrylamide/bisacrylamide gels (Criterion Gels, Bio-Rad Laboratories GmbH, Vienna, Austria). An aliquot (10 µl) of the reaction is mixed with 1 µl loading dye (Elchrom Scientific, Cham, Switzerland) and transferred into one well of the gel each. Similarly at least one well of each gel is used for a molecular weight marker (100 bp Molecular Ruler, Bio-Rad Laboratories GmbH, Vienna, Austria). Electrophoresis is performed at 130 V for 45 min followed by an ethidiumbromide staining (1 µg/ml; Bio-Rad Laboratories GmbH, Vienna, Austria) for 10 min. Bands of PCR products can be visualised on a transilluminator under UV-Light (Gel documentation system, Genxpress, Wiener Neudorf, Austria; see Figure 2). Depending on the intensity of the respective bands 10-20 µl of PCR product is used for further analysis.

### 5. Purification of PCR products

To remove excess primers and dNTPs, 10-20 µl of each PCR product are purified with the illustra GFX™ PCR DNA and Gel Band Purification Kit (GE Healthcare, Munich, Germany).

### 6. Sequence analysis

For sequence analysis the BigDye Terminator Cycle Sequencing Kit (Applied Biosystems, Foster City, CA) is used according to the manufacturer's instructions. The reaction volume is 5 µl containing 1 µl Reaction Mix, 0.5 to 1 µl sample and 2 pmol primer. The standard cycling profile is applied - 25x (96°C 10s, 50°C 5s, 60°C 180s). Separate reactions have to be set up for each primer used. Excess dye-labelled terminators are removed using Centri-Sep spin columns (Applied Biosystems, Foster City, CA). Briefly, the column gel is hydrated with pure water (Merck, Darmstadt, Germany) at room temperature for 2 hours and spun in a microcentifuge at 750g for 2 min to remove the interstitial fluid. The sample is mixed with 15 µl pure water (Merck, Darmstadt, Germany), applied to the column and spun again. The filtrate is added to 20 µl Hi-Di™ formamide (Applied Biosystems, Foster City, CA) for loading to the instrument. Separation and analysis of the sequencing reaction products are performed on an ABI Prism^{®} 310 Genetic Analyzer or an Applied Biosystems 3130 Genetic Analyzer using standard protocols (see Table 13).

**Table 12: Capillary electrophoresis**

| Instrument | capillary length | polymer type | program |
|---|---|---|---|
| 310 | 47 cm | POP6 | ABI Prism™ 310 Collection v1.2.2 |
| 3130 | 50 cm | POP6 | 3130 Data Collection v3.0 |
| Applied Biosystems, Foster City, CA | | | |

### 7. Detection of sequence variants (mutations)

Sequence curves obtained from the analysis of different samples are aligned with the Autoassembler v2.1 or SeqScape v2.6 program (Applied Biosystems, Foster City, CA) and visually compared by a trained person. Sequence variants are detected by the appearance of more than one peak at one position in one sample compared to others as well as to the reference sequence (Accession no.: U94788).

### 8. Quality control issues

### 8.1 Quality and purity of PCR amplification products

Each PCR reaction setup is used without DNA to detect possible contaminations in any reagent used. To detect contaminations and to inspect amount and size of the respective fragments PCR amplification products are visualised after polyacrlyamid-gel electrophoresis.

### 8.2 Method for detection of mutations

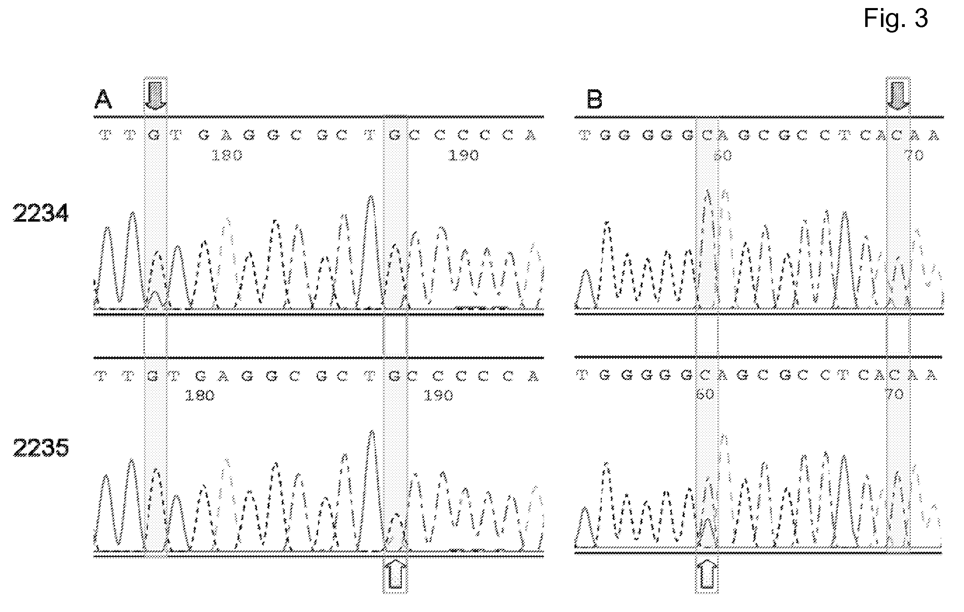

Visual inspection of sequence curves by an experienced person is currently the most reliable method to detect DNA variants. Visual detection of sequence variants is always done by comparison of sequence curves from different samples to the reference sequence (Accession no.: U94788). Results are confirmed by sequencing using forward and reverse primers after PCR amplification in triplicates. The impact of evaluating sequencing curves of both strands has recently been shown by Li et al. (Hum. Mutat. 30 (2009), 1583-1590). The mutation displayed in the supplemental figure S10 of this article (corresponds to Fig. 3 herein) is only visible in the forward, but not in the reverse strand. However, as no sequence curves of other samples were given in this publication, it could not be ruled out that a reduced height of the respective normal peak would have indicated the mutation, as shown in Fig. 3 (B) herein where the mutation in sample 2234 is barely visible, but the peak height is lower than normal as shown in sample 2235 (hatched arrow).

### 9. Performance of the method according to the present invention:

To evaluate several samples of a study sequencing traces from forward and reverse strand sequencing of two or more samples and the respective parts of the published reference sequence with the accession number U94788 are aligned as outlined in Fig. 3 (the maximum number of samples that can be evaluated simultaneously depends on the programme used and on the performance of the computer). Sequence curves are visually inspected to detect differences in the peak pattern. Samples can mutually serve as normal controls as long as they have mutations at different sites. Differences in the peak pattern are described according to the nomenclature for the description of sequence variants (http://www.hgvs.org/mutnomen/; den Dunnen & Antonarakis, Human Mutation 15 (2000) 7-12). In the manner specified all sequences from the triplicate PCR amplifications generated by forward and reverse strand sequencing of each exon are evaluated. This evaluation results in the classification of each sample as normal, if no difference to the reference sequence is detected, or mutated in case of a difference which is not listed as polymorphism e.g. in the IARC p53 database (http://www-p53.iarc.fr).

### 10. General remarks

The advantages of the method according to the present invention are specifically pronounced when the test is applied in connection with the p53 qualitative interaction. These advantages are due to the combination of a quality-controlled, triplicate multiplex PCR as disclosed herein and the automated sequencing using forward and reverse primers for sequencing for all amplicons of the triplicate set-up and, preferably, the visual inspection of the sequence.

Already the primer design in the PCR according to the present invention is diligently performed. The primer set as disclosed in the example section of the present application have provided strikingly good and reliable results. However, when respecting general rules of primer design, primers can be also positioned differently from the position selected in the present examples, but the following issues have to be considered:
- the amplicon must include the whole exon including splice sites
- annealing temperature has to be similar for primers intended to be multiplexed
- a multiplex reaction must not include overlapping amplicons
- amplicons of a multiplex reaction must differ in size to allow quality check by gel electrophoresis
- amplicons should be as short as possible to allow amplification from difficult samples with a high degree of DNA degradation (e.g. formalin-fixed, paraffinised tissues)

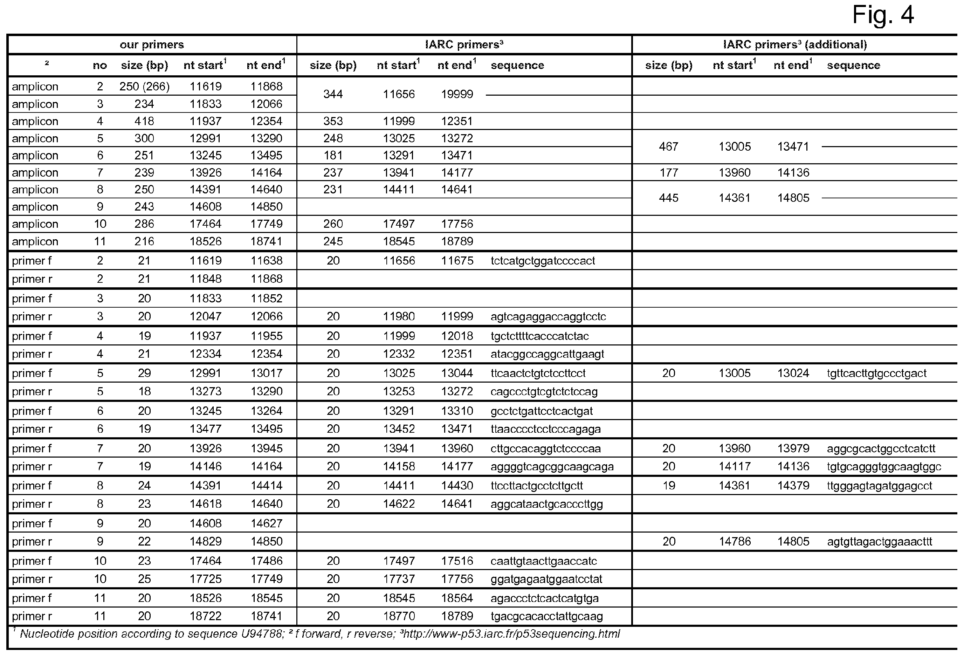

A comparison of primers used in the present example section to those used by the IARC sequencing service is given in Fig. 4. Generally most primers from the IARC are located closer to the exon sequence than those of the present examples. Exons three and nine are analysed together with the respective preceding exon which results in large amplicons and may lead to insufficient amplification from difficult DNA samples.

As an example, primer design of exon 4 is shortly described: Intron 3 is quite short (115 bp) and contains a repetitive sequence stretch which impairs selection of a position for the forward primer. Most groups as well as the IARC selected a primer position close to the exon sequence (IARC uses 2 bp distance).

Due to technical reasons of DNA sequencing using the Sanger method, the first 5-20 bp after the primer cannot be evaluated with sufficient reliability for mutation detection. Especially when using the same primer for PCR amplification and sequencing this limitation is eminent. To circumvent this problem a PCR primer located adjacent to the repetitive sequence and a sequencing primer with 10 bp distance to the exon start is used according to the present invention. With this combination it is possible to reliably analyse at least two bp before the exon start using the forward primer and the whole intronic sequence up to the repeat with the reverse primer.

Mutation classification: The results of DNA sequencing may comprise changes of nucleotides. From these changes together with general knowledge of protein expression (mechanisms of translation), the impact on the function of the protein can be deduced. A base exchange at a certain position may create a stop codon, lead to the usage of another amino acid at this position or produce no apparent alteration. All these changes happen at the level of translation, but this base exchange may be already effective in mRNA processing. A translationally silent mutation may produce or disrupt a splice site as well as a binding site for regulatory factors (proteins, microRNAs). Furthermore very little is known about the relevance of intronic variants. Concerning splice site alterations a number of calculation programs are available to support the detection of a newly created or disrupted site (e.g.: Reese et al., J. Comp. Biol. 4 (1997), 311-323; Heebsgard et al., NAR 24 (1996), 3439-3452)). However, as the mechanism of splicing has not been resolved completely, none of these programs reflect the complete range of possible effects in vivo. It is widely accepted that a change within two base-pairs from the intron-exon-border impairs splicing and it is presumed to be also valid for the region of five base-pairs in each direction.

Based on these arguments all mutations qualify as functionally relevant in some way, unless they are known polymorphisms. Polymorphisms are present at a certain frequency in a population and have no (within an average lifespan of man not obvious) functional impact. Currently 85 polymorphisms in the p53 gene are listed in the IARC p53 database (http://www-p53.iarc.fr/PolymorphismView.asp).

Chemicals and kits used: PCR chemicals and enzymes are not restricted to those used in the present examples, but reaction conditions have to be optimised to obtain pure amplification products free of side products.

Quality control of PCR amplification can be done with any manual or automatic electrophoresis system available. Clean-up of PCR and sequencing products can be done using other methods and kits if quality of the result is provided. Sequence reaction and analysis can be transferred to systems from other supplier (e.g. Beckmann-Coulter CEQ) if the signal to noise ratio is adequate to detect variants in samples with a relatively high amount of normal DNA.

## Claims

1. Method for predicting negative consequences of a treatment of a tumour patient with a therapy inducing p53 dependent apoptosis or a therapy interfering with the cell cycle **characterised by** the following steps:
- determining the genetic status of the patient's tumour with respect to p53 by determining in a sample of body fluid or a tissue sample of the patient containing tumour cells or cell-free tumour DNA whether the whole p53 gene is present in native form or whether the p53 gene has one or more mutations;
- predicting the tumour patient as
- (i) a patient who will suffer negative consequences of a therapy interfering with the cell cycle if the whole p53 gene is present in native form; or
- (ii) a patient who will suffer negative consequences of a therapy inducing p53 dependent apoptosis if the p53 gene has one or more mutations,
wherein
the therapy inducing p53 dependent apoptosis is
- a treatment with methotrexate, 5-fluorouracil, capecitabine, gemcitabine or hydroxyurea;
- a treatment with actinomycin D or anthracyclins, especially doxorubicin, daunorubicin, idarubicin, valrubicin, mitoxantrone or epirubicin;
- a treatment with melphalan, oxazaphosphorins, especially cyclophosphamide, ifosfamide or busulfan; nitrosourea, especially carmustine, lomustine, semustine or procarbazine; or a treatment with cisplatin, carboplatin or oxaliplatin;
- a treatment with thymidylate synthase inhibitors, especially raltitrexed or pemetrexed;
- radiotherapy;
- a treatment with estrogens, gestagens, anti-estrogens, especially tamoxifen, 3-hydroxy-tamoxifen, or chlortamoxifen; aromatase inhibitors, especially aminoglutethimide, formestan, anastrozol or letrozol; antiandrogens, especially cyproterone acetate or flutamide; gonadotropin-releasing hormone antagonists (buserelin, goserelin, leuprolerin, triptorelin); or
- a treatment with antitumour antibodies, preferably a treatment with a HER2 inhibitor antibody, especially trastuzumab; with a thymidine kinase inhibitor antibody, especially gefitinib or erlotinib; with a EGFR inhibitor antibody, especially cetuximab, panitumumab or nimotuzumab;
and wherein
the therapy interfering with the cell cycle is
- a treatment with camptothecins, especially irinotecan or topotecan;
- a treatment with vinca alcaloids, especially vincristine, vinblastine, vindesine or vinorelbine;; or a treatment with taxanes, especially paclitaxel or docetaxel.

2. Method according to claim 1 **characterised in that** the sample of body fluid or a tissue sample of the patient is a blood sample or a tumour biopsy sample.

3. Method according to claim 1 or 2, **characterised in that** the tumour patient has a solid tumour, preferably colorectal cancer, esophagus cancer, gallbladder cancer, lung cancer, breast cancer, oral cancer, ovarian cancer, pancreas cancer, rectal cancer, gastrointestinal cancer, stomach cancer, liver cancer, kidney cancer, head and neck cancer, cancer of the nervous system, retinal cancer, non-small cell lung cancer, brain cancer, soft tissue cancer, lymphnode cancer, cancer of the endocrine glands, bone cancer, cervix cancer, prostate cancer or skin cancer; or a haematological tumour, preferably acquired aplastic anaemia, myelodysplastic syndrome, acute myeloid leukaemia, acute lymphatic leukaemia, Hodgkin lymphoma, non-Hodgkin lymphoma or multiple myeloma.

## Patentansprüche

1. Verfahren zum Prognostizieren von negativen Folgen einer Behandlung eines Tumorpatienten mit einer p53-abhängige Apoptose induzierenden Therapie oder einer in den Zellzyklus eingreifenden Therapie, **gekennzeichnet durch** die folgenden Schritte:
- Bestimmen des genetischen Status des Tumors des Patienten in Bezug auf p53 durch Bestimmen in einer Probe von Körperflüssigkeit oder einer Gewebeprobe des Patienten, die Tumorzellen oder zellfreie Tumor-DNA enthält, ob das gesamte p53-Gen in nativer Form vorliegt oder ob das p53-Gen eine oder mehrere Mutationen aufweist;
- Prognostizieren des Tumorpatienten als
- (i) einen Patienten, der an negativen Folgen aus einer in den Zellzyklus eingreifenden Therapie leiden wird, wenn das gesamte p53-Gen in nativer Form vorhanden ist; oder
- (ii) einen Patienten, der an negativen Folgen aus einer p53-abhängige Apoptose induzierenden Therapie leiden wird, wenn das p53-Gen eine oder mehrere Mutationen aufweist,
wobei
die p53-abhängige Apoptose induzierende Therapie ist:
- eine Behandlung mit Methotrexat, 5-Fluoruracil, Capecitabin, Gemcitabin oder Hydroxyharnstoff;
- eine Behandlung mit Actinomycin D oder Anthracyclinen, insbesondere Doxorubicin, Daunorubicin, Idarubicin, Valrubicin, Mitoxantron oder Epirubicin;
- eine Behandlung mit Melphalan, Oxazaphosphorinen, insbesondere Cyclophosphamid, Ifosfamid oder Busulfan; Nitrosoharnstoff, insbesondere Carmustin, Lomustin, Semustin oder Procarbazin; oder eine Behandlung mit Cisplatin, Carboplatin oder Oxaliplatin;
- eine Behandlung mit Thymidylatsynthase-Inhibitoren, insbesondere Raltitrexed oder Pemetrexed;
- Radiotherapie;
- eine Behandlung mit Östrogenen, Gestagenen, Anti-Östrogenen, insbesondere Tamoxifen, 3-Hydroxytamoxifen oder Chlortamoxifen; Aromatase-Inhibitoren, insbesondere Aminoglutethimid, Formestan, Anastrozol oder Letrozol; Antiandrogenen, insbesondere Cyproteronacetat oder Flutamid; Gonadotropinfreisetzendes Hormon-Antagonisten (Buserelin, Goserelin, Leuprolerin, Triptorelin); oder
- eine Behandlung mit Antitumor-Antikörpern, vorzugsweise eine Behandlung mit einem HER2-Inhibitor-Antikörper, insbesondere Trastuzumab; mit einem Thymidinkinase-Inhibitor-Antikörper, insbesondere Gefitinib oder Erlotinib; mit einem EGFR-Inhibitor-Antikörper, insbesondere Cetuximab, Panitumumab oder Nimotuzumab;
und wobei
die in den Zellzyklus eingreifende Therapie ist:
- eine Behandlung mit Camptothecinen, insbesondere Irinotecan oder Topotecan;
- eine Behandlung mit Vinca-Alkaloiden, insbesondere Vincristin, Vinblastin, Vindesin oder Vinorelbin; oder eine Behandlung mit Taxanen, insbesondere Paclitaxel oder Docetaxel.

2. Verfahren gemäß Anspruch 1, **dadurch gekennzeichnet, dass** die Probe von Körperflüssigkeit oder eine Gewebeprobe des Patienten eine Blutprobe oder eine Tumorbiopsie-Probe ist.

3. Verfahren gemäß Anspruch 1 oder 2, **dadurch gekennzeichnet, dass** der Tumorpatient einen festen Tumor, vorzugsweise Dickdarmkrebs, Speiseröhrenkrebs, Gallenblasenkrebs, Lungenkrebs, Brustkrebs, Mundhöhlenkrebs, Eierstockkrebs, Bauchspeicheldrüsenkrebs, Mastdarmkrebs, Magen-Darm-Krebs, Magenkrebs, Leberkrebs, Nierenkrebs, Kopf-Hals-Krebs, Krebs des Nervensystems, Netzhautkrebs, nicht-kleinzelligen Lungenkrebs, Hirnkrebs, Weichgewebekrebs, Lymphknotenkrebs, Krebs der endokrinen Drüsen, Knochenkrebs, Gebärmutterhalskrebs, Prostatakrebs oder Hautkrebs; oder einen hämatologischen Tumor, vorzugsweise erworbene aplastische Anämie, myelodysplastisches Syndrom, akute myeloide Leukämie, akute lymphatische Leukämie, Hodgkin-Lymphom, Non-Hodgkin-Lymphom oder multiples Myelom, aufweist.

## Revendications

1. Procédé pour prédire les conséquences négatives d'un traitement d'un patient souffrant d'une tumeur par une thérapie induisant une apoptose dépendante de p53 ou une thérapie interférant avec le cycle cellulaire, **caractérisé par** les étapes suivantes :
- la détermination du statut génétique de la tumeur du patient par rapport à p53 en déterminant, dans un échantillon de fluide corporel ou un échantillon de tissu du patient contenant des cellules tumorales ou de l'ADN tumoral dépourvu de cellules, si le gène p53 entier est présent sous une forme native ou si le gène p53 comporte une ou plusieurs mutations ;
- la prédiction du patient souffrant d'une tumeur en tant que
- (i) patient qui souffrira des conséquences négatives d'une thérapie interférant avec le cycle cellulaire si le gène p53 entier est présent sous une forme native ; ou
- (ii) patient qui souffrira des conséquences négatives d'une thérapie induisant une apoptose dépendante de p53 si le gène p53 comporte une ou plusieurs mutations,
dans lequel
la thérapie induisant une apoptose dépendante de p53 est
- un traitement par le méthotrexate, le 5-fluorouracile, la capécitabine, la gemcitabine ou l'hydroxyurée ;
- un traitement par l'actinomycine D ou des anthracyclines, particulièrement la doxorubicine, la daunorubicine, l'idarubicine, la valrubicine, la mitoxantrone ou l'épirubicine ;
- un traitement par le melphalan, des oxazaphosphorines, particulièrement le cyclophosphamide, l'ifosfamide ou le busulfan ; une nitrosourée, particulièrement la carmustine, la lomustine, la sémustine ou la procarbazine ; ou un traitement par le cisplatine, le carboplatine ou l'oxaliplatine ;
- un traitement par des inhibiteurs de la thymidylate synthase, particulièrement le raltitrexed ou le pémétrexed ;
- la radiothérapie ;
- un traitement par des oestrogènes, des gestagènes, des anti-oestrogènes, particulièrement le tamoxifène, le 3-hydroxy-tamoxifène, ou le chlorotamoxifène ; des inhibiteurs de l'aromatase, particulièrement l'aminoglutéthimide, le formestane, l'anastrozole ou le létrozole ; des anti-androgènes, particulièrement l'acétate de cyprotérone ou le flutamide ; des antagonistes de l'hormone de libération des gonadotrophines (buséréline, goséréline, leuproréline, triptoréline) ; ou
- un traitement par des anticorps antitumoraux, de préférence un traitement par un anticorps inhibiteur de HER2, particulièrement le trastuzumab ; par un anticorps inhibiteur de la thymidine kinase, particulièrement le géfitinib ou l'erlotinib ; par un anticorps inhibiteur de l'EGFR, particulièrement le cétuximab, le panitumumab ou le nimotuzumab ;
et dans lequel
la thérapie interférant avec le cycle cellulaire est
- un traitement par des camptothécines, particulièrement l'irinotécan ou le topotécan ;
- un traitement par des vinca-alcaloïdes, particulièrement la vincristine, la vinblastine, la vindésine ou la vinorelbine ; ou un traitement par des taxanes, particulièrement le paclitaxel ou le docétaxel.

2. Procédé selon la revendication 1, **caractérisé en ce que** l'échantillon de fluide corporel ou un échantillon de tissu du patient est un échantillon de sang ou un échantillon de biopsie tumorale.

3. Procédé selon la revendication 1 ou 2, **caractérisé en ce que** le patient souffrant d'une tumeur présente une tumeur solide, de préférence le cancer colorectal, le cancer de l'oesophage, le cancer de la vésicule biliaire, le cancer du poumon, le cancer du sein, le cancer de la bouche, le cancer de l'ovaire, le cancer du pancréas, le cancer du rectum, le cancer gastro-intestinal, le cancer de l'estomac, le cancer du foie, le cancer du rein, le cancer de la tête et du cou, le cancer du système nerveux, le cancer de la rétine, le cancer du poumon non à petites cellules, le cancer du cerveau, le cancer des tissus mous, le cancer des ganglions lymphatiques, le cancer des glandes endocrines, le cancer des os, le cancer du col de l'utérus, le cancer de la prostate ou le cancer de la peau ; ou une tumeur hématologique, de préférence une anémie aplasique acquise, le syndrome myélodysplasique, la leucémie aiguë myéloïde, la leucémie aiguë lymphoblastique, le lymphome hodgkinien, le lymphome non hodgkinien ou le myélome multiple.


### SEQUENCE LISTING

<110> Kandioler, Daniela Prof.
<120> Response Prediction in Cancer Treatment (p53 Adapted Cancer Therapy)
<130> R 55960
<160> 24
<170> PatentIn version 3.5
<210> 1
   <211> 20303
   <212> DNA
   <213> Homo sapiens
<400> 1
   ttcccatcaa gccctagggc tcctcgtggc tgctgggagt tgtagtctga acgcttctat      60
   cttggcgaga agcgcctacg ctccccctac cgagtcccgc ggtaattctt aaagcacctg     120
   caccgccccc ccgccgcctg cagagggcgc agcaggtctt gcacctcttc tgcatctcat     180
   tctccaggct tcagacctgt ctccctcatt caaaaaatat ttattatcga gctcttactt     240
   gctacccagc actgatatag gcactcagga atacaacaat gaataagata gtagaaaaat     300
   tctatatcct cataaggctt acgtttccat gtactgaaag caatgaacaa ataaatctta     360
   tcagagtgat aagggttgtg aaggagatta aataagatgg tgtgatataa agtatctggg     420
   agaaaacgtt agggtgtgat attacggaaa gccttcctaa aaaatgacat tttaactgat     480
   gagaagaaag gatccagctg agagcaaacg caaaagcttt cttccttcca cccttcatat     540
   ttgacacaat gcaggattcc tccaaaatga tttccaccaa ttctgccctc acagctctgg     600
   cttgcagaat tttccacccc aaaatgttag tatctacggc accaggtcgg cgagaatcct     660
   gactctgcac cctcctcccc aactccattt cctttgcttc ctccggcagg cggattactt     720
   gcccttactt gtcatggcga ctgtccagct ttgtgccagg agcctcgcag gggttgatgg     780
   gattggggtt ttcccctccc atgtgctcaa gactggcgct aaaagttttg agcttctcaa     840
   aagtctagag ccaccgtcca gggagcaggt agctgctggg ctccggggac actttgcgtt     900
   cgggctggga gcgtgctttc cacgacggtg acacgcttcc ctggattggg taagctcctg     960
   actgaacttg atgagtcctc tctgagtcac gggctctcgg ctccgtgtat tttcagctcg    1020
   ggaaaatcgc tggggctggg ggtggggcag tggggactta gcgagtttgg gggtgagtgg    1080
   gatggaagct tggctagagg gatcatcata ggagttgcat tgttgggaga cctgggtgta    1140
   gatgatgggg atgttaggac catccgaact caaagttgaa cgcctaggca gaggagtgga    1200
   gctttgggga accttgagcc ggcctaaagc gtacttcttt gcacatccac ccggtgctgg    1260
   gcgtagggaa tccctgaaat aaaagatgca caaagcattg aggtctgaga cttttggatc    1320
   tcgaaacatt gagaactcat agctgtatat tttagagccc atggcatcct agtgaaaact    1380
   ggggctccat tccgaaatga tcatttgggg gtgatccggg gagcccaagc tgctaaggtc    1440
   ccacaacttc cggacctttg tccttcctgg agcgatcttt ccaggcagcc cccggctccg    1500
   ctagatggag aaaatccaat tgaaggctgt cagtcgtgga agtgagaagt gctaaaccag    1560
   gggtttgccc gccaggccga ggaggaccgt cgcaatctga gaggcccggc agccctgtta 1620
   ttgtttggct ccacatttac atttctgcct cttgcagcag catttccggt ttctttttgc 1680
   cggagcagct cactattcac ccgatgagag gggaggagag agagagaaaa tgtcctttag 1740
   gccggttcct cttacttggc agagggaggc tgctattctc cgcctgcatt tctttttctg 1800
   gattacttag ttatggcctt tgcaaaggca ggggtatttg ttttgatgca aacctcaatc 1860
   cctccccttc tttgaatggt gtgccccacc ccccgggtcg cctgcaacct aggcggacgc 1920
   taccatggcg tagacaggga gggaaagaag tgtgcagaag gcaagcccgg aggcactttc 1980
   aagaatgagc atatctcatc ttcccggaga aaaaaaaaaa agaatggtac gtctgagaat 2040
   gaaattttga aagagtgcaa tgatgggtcg tttgataatt tgtcgggaaa aacaatctac 2100
   ctgttatcta gctttgggct aggccattcc agttccagac gcaggctgaa cgtcgtgaag 2160
   cggaaggggc gggcccgcag gcgtccgtgt ggtcctccgt gcagccctcg gcccgagccg 2220
   gttcttcctg gtaggaggcg gaactcgaat tcatttctcc cgctgcccca tctcttagct 2280
   cgcggttgtt tcattccgca gtttcttccc atgcacctgc cgcgtaccgg ccactttgtg 2340
   ccgtacttac gtcatctttt tcctaaatcg aggtggcatt tacacacagc gccagtgcac 2400
   acagcaagtg cacaggaaga tgagttttgg cccctaaccg ctccgtgatg cctaccaagt 2460
   cacagaccct tttcatcgtc ccagaaacgt ttcatcacgt ctcttcccag tcgattcccg 2520
   accccacctt tattttgatc tccataacca ttttgcctgt tggagaactt catatagaat 2580
   ggaatcagga tgggcgctgt ggctcacgcc tgcactttgg ctcacgcctg cactttggga 2640
   ggccgaggcg ggcggattac ttgaggatag gagttccaga ccagcgtggc caacgtggtg 2700
   aatccccgtc tctactaaaa aatacaaaaa ttagctgggc gtggtgggtg cctgtaatcc 2760
   cagctattcg ggagggtgag gcaggagaat cgcttgaacc cgggaggcag aggttgcagt 2820
   gagccaagat cgtgccacta cactccagcc tgggcgacaa gaacgaaact ccgtctcaaa 2880
   aaaaaggggg gaatcataca ttatgtgctc atttttgtcg ggcttctgtc cttcaatgta 2940
   ctgtctgaca ttcgttcatg ttgtatatat cagtattttg ctccttttca tttagtatag 3000
   tccatcgatt gtatatccgt ccttttgatg gccttttgag ttgtttccca tttgcggtta 3060
   tgaaataaag ctgctataaa cattcttgta caattctttt tgtgatcata tgttttcgtg 3120
   tttcttggag aaatacttag gaggggaatt gtggaggaag taaaaagtag ctgtattttg 3180
   aactttttca gaagctctga gttttccaga gcggttgtac cattttacac tccaactagc 3240
   aaggtatggg agttattatg gttgtgccac agccttccgg acattaggta tgtcagtctt 3300
   tctaatgtgg tatatccttg tggttgtaat ttacagttct ctattgacta aggatgttca 3360
   gcattttttc atgtgcctat tggccattcg tattttgttt gtaaagtagc tcttcgagtc 3420
   ttttacctgt tattttggtt tttttgtttg tttttattgt tcagttgtgg gactgcttta 3480
   tactttctgg atacaagtcc ttatcagatc catgagtcgt gaatgttttc ttctgatctg 3540
   ttgcgggcct atttgtttgc tttacagagt ttacagaatc ttaagaggag tggattaatc 3600
   ttttttatgt tcagtatttg ccttgtcctg tttaggacat cttttttttt ttttttaacc 3660
   ccagggtcat gaagatataa tcttacattt tcttttagga cctttatggt ggtaagtttt     3720
   acagtaaggt ccttaagcca ttaattaatt cttaaaatta attgtttatg gtgtgaggtg     3780
   taggagtcag tctctggtat ctttcctgta tggaaatcca gttattctgt ctccacttgt     3840
   tgaaataggc ttcctttctc tactgaatgc ttttaatttt aattatttta cagttggagt     3900
   atagggctac cattttagtg ctattttctt tttttctttg ttaatttttg agacagggac     3960
   tcacactgtt gcccaggcta gagtacaatg gcacaatcaa ggcttactgc agcctcgaac     4020
   ccctgggctc aagcagtcct ctagcagcct cacgagtagc tgggattact ccaccacacc     4080
   cagctaacta ttttattttt ttgtattgac aggatctcac tatgttgccc aggctggtct     4140
   caaactgctg gcctcaagct ttcatcccat ctcggcctcc caaagtgctg ggattacagg     4200
   tgtgagccac catgcctgac ctcttagtgc tattttctat ttatctcctc tgttctctgc     4260
   tctctttaaa cgttggagga agaaacagta cccatcttac acaaactctt cagaaaacag     4320
   aggaacagac tgggcgcggt ggctcatacc tgtaatctca gcactttggt acgctgaggc     4380
   aggggatcat ttgaggtcgg gagttcgaga ccagcctggc caacacggcg aaaccccatc     4440
   tctactaaaa tacaaaagta gctaggcgtg caccatacct gtaatgccag ttactcagga     4500
   ggctgaggca caagaatccc ttgaacctgg gaagcggagg ttgcagtgag ccgagattgc     4560
   gccactgcac tccagcctgg gcaacagagt gagaccctgt ctcagaaaaa aaaagaaaga     4620
   aagaaaaaat agaggaatat ttcccaactt gttttcgaag ccaggataat cctggtacca     4680
   aaaccaaaca aggacattat aagaaaagaa aatatagacc aatattcctg ttagcataga     4740
   catgcaacag ctaaccaatt ttagcaaacc aaacctggta atatagaaaa aaggataaat     4800
   aggccagtcg cggtggctca cgcctgtaat cccagcactt tgggaggctg aggcaggcag     4860
   atcacttgag gtcaggagtt tgagaccagc ctgaccaaca tggtgaaacc ccgtttctaa     4920
   taaaaataca aaaatcaggc tgggcacggt ggctcacgcc tgtaatccca gcactttggg     4980
   aggccgaggt gggcagatca cgaggtcagg agttcaagac cagcctgacc aatgtggtga     5040
   aacgccatct ctactaaaaa tacgaaaatc agccggtgtg gtggcacctg cctgtaatcc     5100
   cagctactca ggaggctgag gcagaattgc ttgaacccgg gaggcagagg ttgcagtgag     5160
   ccaagatcgt gccactgcac tccagcctgg gcgacagagc aagacttcat ctcaaaaaaa     5220
   aaaaaaatta gctgggcatg gtggtgggca cctgaaatcc cagctactcg ggagtctgag     5280
   gcaggagaat cgcttgaacc caggaggcag aagttgcact gagctgggat cacaccattg     5340
   cactccagcc tgggcaacag agtgagactc catctcaaaa aaagaaaaag aaaaaggata     5400
   aatacattct aaccaaataa tgtttatctc atgattgtag ctgattcaac attcaaaaat     5460
   tggcctggtg cagtagctca ggcctgtaat cccaacattt taggaggctg aggcaggaag     5520
   atctcttgag cccaggattt caagaccagc ctgggcaaca tagtcagact ggtctttact     5580
   ggggggaaaa aaatcagtct gtgtaattca ccacattaac aaagggaaac ataaaaaccc     5640
   tatgatcatt tcaacagatg tagcaaaagc agttaatgat atcaacacat atgcatgatt     5700
   acaaaccaac caacctccta gcaaactagg gaaaggaaac ttaactagtt tgataacagg     5760
   gcgtccacag tcggagttcc actagcagca tacataatgg tagaaaactc agtgctgctg 5820
   ggggcggtgg ctcacgcctg taatgccagc gctttgggag gcctaggcgg gcggatcacg 5880
   aggtcaggag atcgagactg tcctgactag catgctgaaa ccccgtctct actaaaaata 5940
   caaaaacaaa aaattagccg ggcatggtgg cgggcgccta tagtgccagc tactcgggag 6000
   gctgaggcga gagaatggcg tgaacccggg aggcggagct tgcagagcct agatcgtgcc 6060
   actgcactcc agcctgggtg acagagtgag acttcgtctc aaaaaaaaaa aaaaaaaaaa 6120
   aagaaaagaa aactcaacgc tttttcctct aagatcagga actagaaaag gatttgactc 6180
   tcacaacgtt gataccatac tggaggtttt aaccaggcaa gaaaaagaaa taatgagggc 6240
   cgggtgcggt ggctcaggcc tgtaatccca gcactttggg aagccgagac gggtggatca 6300
   cgaggtcagg agatcgagcc atcctggcta acacggtgaa accctgtctc tactaaatat 6360
   acaaaaaatt agccgggcgt ggtggcgggc gcctgtagtc ccagctactc gggaggctga 6420
   ggcaggagaa tggcgtgaac tcagggggcg gagcttgcag tgagctgaga tcgagccact 6480
   gcactccagc ctgggcgaca gagcaagact gtgtctcaaa aaaaaaaaaa gaaaaagaaa 6540
   taatgattag tggcccgatg tctcacgcca gtaatcccag cactttggga ggccgaggtg 6600
   ggcagatcac ctgaggtctg gagttggaga ccagcctgac aaagatggtg aaacctcgtc 6660
   tctattaaaa tattaaaaaa atagccaggc gttggccggg tacagtggct catgcctgta 6720
   accccagcac tttgggaggc cgaggtgggt ggatcacctg aggtcaggag ttcaacacca 6780
   gcctggccaa catggtgaaa ccccatctct actaaaaata caaaattagc cgggcgtagt 6840
   ggcgggcgcc tgtaatccca gctacttggg aggcttaggc aggagaatcg cttgaacctg 6900
   ggaggcggag gttgtagtga gccgagattg caccattgca ctccagcctg ggtgacaaaa 6960
   gcaaaaactc cgtctcaaaa aaaaaagaat tagccagggg tagtggtgaa cgcctgtagt 7020
   cccagctact caggaggcag aggcaggaga atcacttgaa ccccggaggc agaggttgca 7080
   gtgagccgag attgtcccat tgcactccag cctaggcgag aagagcaaaa ttccatgtca 7140
   aaaaaaaaaa aaaaaaagga aagaaaaaaa ataacgatta gaaaggaaga aatcaaacac 7200
   attcacagcc agtatgattc tatacatacc atggtcctaa tggggccagg cgtggtggct 7260
   catgctgtaa tcctagcact tttaggaggc tgaggcaggt ggcttccctg ggaccagctg 7320
   gccaacatgg tgaaacccca actctaataa aaatacaaaa aatcagccag gcgtggtgag 7380
   ggcacctcta atcccagcta ctcaggaggc tgaggcagga gaattgcttg gacctgggag 7440
   gcagaggttg cagtgagccg agatcgcgct attgcactcc agcctgggca acaagagtga 7500
   aactccggca gggtgtggtc ttacgcctgt aatcccagca cttcgggagg ctgagccagg 7560
   ccgatcacct gaggtcagga gtttgagacc aacctaacat ggtgaaaccc cgtctctact 7620
   aaaaatacaa gaattagctg ggtgtagtgg tgggcgcctg taatcccagc tacttgggag 7680
   gctgagacag aagaattgct tgaacccagg aggtggaggt tgcagtgagc tgagatcatg 7740
   ccattgcaca ccacgccggg caacagagcg agattccgtc tcaaaaaaaa aaaaaagatg 7800
   aaactctatc tcaaaaaaaa aaaaaagtcc taatggaaaa tccataaaaa gctaccaaaa 7860
   ctaataaata aatatagcag ggttgcaggt tacagggcaa tatagttatc cctctatctg 7920
   taggggcttg gttctgggac tcctcacaca ccaaacccac agatgtctaa gtcccatata 7980
   taagacggaa tagtatttaa cctacacata tcctcccata tagtttaaat tatctagatt 8040
   acttacatta cccccataca atgaaaatgc taatgtacat gcaagtatgt atgtaagtac 8100
   ttgtactata ttgtttaggg aatcactgga cagataggcc ttcaagactg ataccagcag 8160
   ccactgttaa gattctggtc aggcctgccc ctgtttgggg tctcagttga tctcattgcc 8220
   ttcccaccca gccaagggca cctgcatttc tcttggctcc ctggccattt ggaaggccta 8280
   gttcagcctg gcacatttgt atcctggccc actgatgctg gtacccctgg gaaggtcctg 8340
   ctctgaaaaa cacggagatt ttagttgcta ctgaagattt gagagataaa gacagggaga 8400
   cctgtctgta gacctgtgtc cctccaagtg ggattgagac tttgggcccc ccatttcagg 8460
   acagcacctc ctggcctgtt gactgaatag atccctgaag gaggtgtagt tgcattttag 8520
   gagtgggggt gggagcagta ccactgatcc gcactaacaa tcacacagtt ctctctagaa 8580
   taataatata gaacaagtga aatagaacaa ttgcagaaag agctaacctt tgttgagctc 8640
   ttactgtgtg cccagcactt tcctcaactc tacatttccc ataatacata gagtactagg 8700
   taggcggggc ttgggggctc acgcctgtaa tcccagcact ttaggaggcc aaggggggtg 8760
   gatcacctga ggtcgggagt tcaagaccag cctgactaac atggtgaaac cccgtctcta 8820
   ctagaagtac aaaattagcc aggtgtggtg gcacatgctt gtagtcctag ctactcagca 8880
   ggctgaggca ggagaatcat ttgaatccgg gaggaggttg cagtaagcgg agatagtgcc 8940
   actgtactcc agcctgggca ataagagctg agactccgtc tcaaaataaa ataaaataaa 9000
   ataaaataaa ataaaataaa ataaaaaaag aaaagagcct gccattaaag gagctgtttg 9060
   gtaggggatg ttttgtcagt gcaaacaaca gaaaagtggg ctgggcacag tggttcatgc 9120
   ctgtaatccc agcactttgg gaggccaagg cgggcggatc acctgaagtt gggagttcaa 9180
   gaccagcctg accaatatgg agaaaccccg tctctactaa aaatacaaaa ttagccgggc 9240
   gcagtggccg atgcctgtaa tcccagctac tcgggaggct gaggcaggag aatcgcttga 9300
   acctgggagg cagaggttgc ggtgagccga gatcgcacca ttgcactcca gcctggacga 9360
   gagcaaaact ctgtctcaaa aaaaaaaaaa aacagaaaag tgtaacaaac acttacagta 9420
   ggcatgtttc ttagcaaatc tgatgacaaa tttggcataa agaaagagag catccctgaa 9480
   aaaaaaaaaa agaaaaagaa agagagcatc ctgcctgggc aacatagtga aaccctgcct 9540
   ctacaaaaaa actcaaaaat tggccgggtg cagtggctca cacctgtaat cccagcactt 9600
   tgggagtcgg aggcgggagg atcacctgag gtcaggagtt cgaaaccagc ctggccaaca 9660
   tggcaaaacc ccatctctac taaaaataca aaaaattaat caggcgcatt ggtgggcgcc 9720
   tgtaatccca gctactcagg aagttgaggc aagaggatcg cttgatactg ggaggtggag 9780
   gttacagtga gtcgagatca caccactgca ctctagcctg ggtgacaggg cgagactccg 9840
   tctccaaaaa aaaaaagaaa aagaaaaaga ctaaaaaatt agccaggcag gcctctgtgg 9900
   tcccagctac ttgggaggct gaggcaggag aatcactgag cccaggagtg gcaggctgta 9960
   gtgagccatg attgcaccac tgtaccctag cttgggcttc aaagcaagac cctgcctcaa 10020
   aagaaaaaag aaagaaagaa agaacatggc gggccaggca cagtggctca cacctgtaat 10080
   cccagcgctt tgagaggccg aggcaggtgg atcacaaggt caggagttcc acaccagcct 10140
   ggccaacatg gtgaaaccct gtctctacta aaaatacaaa aaatcagcag gcagggtggt 10200
   aggggcctgt aatcccagct actcgggagg ctgaggcagg agaattgctt gaaaccagaa 10260
   ggcagaggtt gcagtgagcc tagactgcac cactgcactc cagcctgggc gaaaagagcc 10320
   aaactccatc tcaaaaaaca aacaaaaaaa caaaacaaaa aaaaacatgg cagcctttga 10380
   aagcttgtct gggagaaggt gcgatgatgg ttgcataact tcgtgcaaga tgctggtcca 10440
   cacaggggct gccccttgct ctttctcgct ctcttaacct ctcatataac aggcttgtgt 10500
   gttatgcaca tttattgagc ccaagcaggt gcaaggcatt gtgatctaat actttggtca 10560
   gcaagacaac aagatagatc actgccctgc ccttaggaag tgtatatgct attagaggaa 10620
   acagataaaa taaacaagga aaagtatcag acaatgtaag tgctatgaga atgcaaatga 10680
   ggtgatgtga attaaaatag gatgacttaa gtctgcacgg aaggccccta cccccatgtt 10740
   cctggctagc caaggaacca ccagttgatt agcagagaag ggcagcccgt ctagctagag 10800
   cttttgggga agagggagtg gttgttaaga gatgagatta aagaagccga gacgggccct 10860
   tcgtgagggg gggttgtaat gcagggctga ggagtgtccg aagagaatgg gcaggtgagc 10920
   ggtgagacag ttgttcttcc agaagctttg cagtgaaagg aatcaaagaa atggagccgt 10980
   gtatcaggtg gggaagggtg ggggccaagg gggtgtcctt ccccatacag agattgcagg 11040
   ctgagaatga ctatatcctt gttaacagga ggtgggagca gggcacggta gctcacacct 11100
   gtaatcttgg cactttagga ggcggaggcg ggccgatcac ctgaagtaag gagttcgaga 11160
   ccagcctggc caacatgcaa agccctgtct ctactaaaaa tacaaaaatt agctgggtgt 11220
   ggtggtactc gcctgtaatc ccagctactc gggagactga ggcaggagaa tggcttgaac 11280
   ccggaaggta gaggttgcag tgagctgaga tcatgccact gtgctccagc ctaggtgaca 11340
   gagagagact ccatctcaaa aaaaaaaaaa aatacaggaa gggagttggg aatagggtgc 11400
   acatttagga agtcttgggg atttagtggt gggaaggttg gaagtccctc tctgattgtc 11460
   ttttcctcaa agaagtgcat ggctggtgtg gggtggggca ggagtgcttg ggttgtggtg 11520
   aaacattgga agagagaatg tgaagcagcc attcttttcc tgctccacag gaagccgagc 11580
   tgtctcagac actggcatgg tgttggggga gggggttcct tctctgcagg cccaggtgac 11640
   ccagggttgg aagtgtctca tgctggatcc ccacttttcc tcttgcagca gccagactgc 11700
   cttccgggtc actgccatgg aggagccgca gtcagatcct agcgtcgagc cccctctgag 11760
   tcaggaaaca ttttcagacc tatggaaact gtgagtggat ccattggaag ggcaggccac 11820
   caccccgacc ccaaccccag ccccctagca gagacctgtg ggaagcgaaa attcatggga 11880
   ctgactttct gctcttgtct ttcagacttc ctgaaaacaa cgttctggta aggacaaggg 11940
   ttgggctggg gacctggagg gctggggggc tggggggctg aggacctggt cctctgactg 12000
   ctcttttcac ccatctacag tcccccttgc cgtcccaagc aatggatgat ttgatgctgt 12060
   ccccggacga tattgaacaa tggttcactg aagacccagg tccagatgaa gctcccagaa 12120
   tgccagaggc tgctccccgc gtggcccctg caccagcagc tcctacaccg gcggcccctg 12180
   caccagcccc ctcctggccc ctgtcatctt ctgtcccttc ccagaaaacc taccagggca 12240
   gctacggttt ccgtctgggc ttcttgcatt ctgggacagc caagtctgtg acttgcacgg 12300
   tcagttgccc tgaggggctg gcttccatga gacttcaatg cctggccgta tccccctgca 12360
   tttcttttgt ttggaacttt gggattcctc ttcaccctta ggcttcctgt cagtgttttt 12420
   ttatagttta cccacttaat gtgtgatctc tgactcctgt cccaaagttg aatattcccc 12480
   ccttgaattt gggcttttat ccatcccatc acaccctcag catctctcct ggggatgcag 12540
   aacttttctt tttcttcatc cacgtgtatt ccttggcttt tgaaaataag ctcctgacca 12600
   ggcttggtgg ctcacacctg caatcccagc actctcaaag aggccaaggc aggcagatca 12660
   cctgagcccc aggagttcaa gaccagcctg ggtaacatga tgaaacctcg tctctacaaa 12720
   aaaatacaaa aaattagcca ggcatggtgg tgcacaccta tagtcccagc cactcaggag 12780
   gctgaggtgg gaagatcact tgaggccagg agatggaggc tgcagtgagc tgtgatcaca 12840
   ccactgtgct ccagcctgag tgacagagca agaccctatc tcaaaaaaaa aaaaaaagaa 12900
   aagctcctga ggtgtagacg ccaactctct ctagctcgct agtgggttgc aggaggtgct 12960
   tacacatgtt tgtttctttg ctgccgtgtt ccagttgctt tatctgttca cttgtgccct 13020
   gactttcaac tctgtctcct tcctcttcct acagtactcc cctgccctca acaagatgtt 13080
   ttgccaactg gccaagacct gccctgtgca gctgtgggtt gattccacac ccccgcccgg 13140
   cacccgcgtc cgcgccatgg ccatctacaa gcagtcacag cacatgacgg aggttgtgag 13200
   gcgctgcccc caccatgagc gctgctcaga tagcgatggt gagcagctgg ggctggagag 13260
   acgacagggc tggttgccca gggtccccag gcctctgatt cctcactgat tgctcttagg 13320
   tctggcccct cctcagcatc ttatccgagt ggaaggaaat ttgcgtgtgg agtatttgga 13380
   tgacagaaac acttttcgac atagtgtggt ggtgccctat gagccgcctg aggtctggtt 13440
   tgcaactggg gtctctggga ggaggggtta agggtggttg tcagtggccc tccgggtgag 13500
   cagtaggggg gctttctcct gctgcttatt tgacctccct ataaccccat gagatgtgca 13560
   aagtaaatgg gtttaactat tgcacagttg aaaaaactga agcttacgag gctaagggcc 13620
   tcccctgctt ggctgggcgc agtggctcat gcctgtaatc ccagcacttt gggaggccaa 13680
   ggcaggcgga tcacgaggtt gggagatcga gaccatcctg gctaacggtg aaaccccgtc 13740
   tctactgaaa aatacaaaaa aaaattagcc gggcgtggtg ctgggcacct gtagtcccag 13800
   ctactcggga ggctgaggaa ggagaatggc gtgaacctgg gcggtggagc ttgcagtgag 13860
   ctgagatcac gccactgcac tccagcctgg gcgacagagc gagattccat ctcaaaaaaa 13920
   aaaaaaaaag gcctcccctg cttgccacag gtctccccaa ggcgcactgg cctcatcttg 13980
   ggcctgtgtt atctcctagg ttggctctga ctgtaccacc atccactaca actacatgtg 14040
   taacagttcc tgcatgggcg gcatgaaccg gaggcccatc ctcaccatca tcacactgga 14100
   agactccagg tcaggagcca cttgccaccc tgcacactgg cctgctgtgc cccagcctct 14160
   gcttgccgct gacccctggg cccacctctt accgatttct tccatactac tacccatcca 14220
   cctctcatca catttccggc gggaatctcc ttactgctcc cactcagttt ccttttctct 14280
   ggctttggga cctcttaacc tgtggcttct cctcccacct cctggagctg gagcttaggc 14340
   tccagaaagg acaagggtgg ttgggagtag atggagcctg gttttttaaa tgggacaggt 14400
   aggacctgat ttccttactg cctcttgctt ctcttttcct atcctgagta gtggtaatct 14460
   actgggacgg aacagctttg aggtgcgtgt ttgtgcctgt cctgggagag accggcgcac 14520
   agaggaagag aatctccgca agaaagggga gcctcaccac gagctgcccc cagggagcac 14580
   taagcgaggt aagcaagcag gacaagaagc ggtggaggag accaagggtg cagttatgcc 14640
   tcagattcac ttttatcacc tttccttgcc tctttcctag cactgcccaa caacaccagc 14700
   tcctctcccc agccaaagaa gaaaccactg gatggagaat atttcaccct tcaggtacta 14760
   agtcttggga cctcttatca agtggaaagt ttccagtcta acactcaaaa tgccgttttc 14820
   ttcttgactg ttttacctgc aattggggca tttgccatca gggggcagtg atgcctcaaa 14880
   gacaatggct cctggttgta gctaactaac ttcagaacac caacttatac cataatatat 14940
   attttaaagg accagaccag ctttcaaaaa gaaaatagtt aaagagagca tgaaaatggt 15000
   tctatgactt tgcctgatac agatgctact tgacttacga tggagttact tctgataact 15060
   cgtcgtaagt tgaaatattg aaatattgta agttgaaaat ggatttaata cacctaatct 15120
   aaggaacatc atagcttagc ctagcctgct tttttttttt tttttttttt ggagacagag 15180
   tctcactctg ctacccaggc tggagtgcag tggcgggatc tcggctcact gcaacctccg 15240
   ccttctgggt tcaagcgatt ctcctgcctc agcccactga gtagctggga ttacaggcac 15300
   ctgccccgac gcccagctaa ttttttgtta tttatttcct ttttttttag tagagataga 15360
   atttcaccat gttggccagg ctagtctcga actcctgacc ttgtgatctg cctgccttgg 15420
   cctcccaaag tgctgggatt acaggcgtga gccaccgcac ctggcctgcc tagcctactt 15480
   ttattttatt tttaatggag acagcatctt gctctgttgc ccaggctgga ttacagtgat 15540
   gtgatcatag ctcattatac cctcctgggc tcaagcaatc cccctaactc tgcctcccca 15600
   gtagctagga ccacaggcat acaccaccat acccagctaa tttttaaaat tttttgtaga 15660
   tagatagagt ctcactatgt tgcccaggct ggtctctagc ctactttttt gagacaaggt 15720
   cttgctctgt cacccaggct ggatagagtg cagtagtgca gtcacagctc actgcagcct 15780
   ccacctccca ggctccatcc atcctcccag ctcagcctcc caagttgctt caactacagg 15840
   cctgcaccac catgcctggc taatttttat ttatttattt ttattttatt ttattttatt 15900
   ttttgagact cagtctcact ctgtcgcctt aggctggagt gcagtggcat gatctcggct 15960
   cactgctaac ctctgcctcc tgggtttcaa gtgattctcc tgcctcagcc tcccgaatag 16020
   ctaggactac aagcgcctgc taccacgccc ggctaatttg tgtattttta gtagagacag 16080
   ggtttcacca tgttggccag gctggtctcg aacttctgac catgtgatcg ccgcctcggc 16140
   ctcccaaagt gctgggatta caggtgtgag ccaccacgcc cggctaattt ttatttattt 16200
   atttaaagac agagtctcac tctgtcactc aggctagagt gcagtggcac catctcagct 16260
   cactgcagcc ttgacctccc tgggctccgg tgatttcacc ctcccaagta gctaggacta 16320
   caggcacatg ccacgacacc cagctaattt tttattttct gtgaagtcaa ggtcttgcta 16380
   cgttgcccat gctggtatca aacccctggg ctcaatcaat ccttccacct cagcctcccc 16440
   aagtattggg gttacaggca tgagctacca cactcagccc tagcctactt gaaacgtgtt 16500
   cagagcattt aagttaccct acagttgggc aaagtcatct aacacaaagc cctttttata 16560
   gtaataaaat gttgtatatc tcatgtgatt tattagatat tgttactaaa agtgagaaac 16620
   agcatggttg catgaaagga ggcacagtcg aagccaggca cagcctgggc gcagagcgag 16680
   actcaaaaaa agaaaaggcc aggcgcactc tcacgcctgt aatcccagca tttcgggagg 16740
   ctgaggcggg tggatcacct gaggtcagga gttcaagacc agcctagcca acatggtgaa 16800
   accccgtctc tactaaaata caaaaattaa ccgggcgtga tggcaggtgc ctgtaatccc 16860
   agctacttgg gaggctgagg caggagaatc gcttgaacca ggaggcggag gttgcaggga 16920
   gccaagacgg cgccactgca ctccagcctg ggcgatagag tgagactccg tctcagaaaa 16980
   aaaagaaaag aaacgaggca cagtcgcatg cacatgtagt cccagttact tgagaggcta 17040
   aggcaggagg atctcttgag cccaagagtt tgagtccagc ctgaacaaca tagcaagaca 17100
   tcatctctaa aatttaaaaa agggccgggc acagtggctc acacctgtaa tcccagcact 17160
   ttgggaggtg gaggtgggta gatcacctga cgtcaggagt tggaaaccag cctggctaac 17220
   atggtgaagc cccatctcta ctaaaaacac aaaaattagc cagtgtgaga cacgttgagt 17280
   ccacgtactc ggaggctgag gcacaagaat cacttgaacc ccagaggcgg agattcgaat 17340
   cagccaagat tgcaccattg cactcccgcc tgggcgacga gagtgagacc ccatctcaaa 17400
   ataaataaat aaatattttt aaaagtcagc tgtataggta cttgaagtgc agtttctact 17460
   aaatcgatgt tgcttttgat ccgtcataaa gtcaaacaat tgtaacttga accatctttt 17520
   aactcaggta ctgtgtatat acttacttct ccccctcctc tgttgctgca gatccgtggg 17580
   cgtgagcgct tcgagatgtt ccgagagctg aatgaggcct tggaactcaa ggatgcccag 17640
   gctgggaagg agccaggggg gagcagggct cactccaggt gagtgacctc agccccttcc 17700
   tggccctact cccctgcctt cctaggttgg aaagccatag gattccattc tcatcctgcc 17760
   ttcatggtca aaggcagctg accccatctc attgggtccc agccctgcac agacattttt 17820
   ttagtcttcc tccggttgaa tcctataacc acattcttgc ctccacgtag tatccacaga 17880
   acatccaaac ccagggacga gtgtggatac ttctttgcca ttctccgcca actccccagc 17940
   ccagagctgg agggtctcaa ggggcctaat aattgtgtaa tactgaatac agccagagtt 18000
   tcaggtcata tactcagccc tgccatgcac cggcaggtcc taggtgaccc ccgtcaaact 18060
   cagtttcctt atatataaaa tggggtaagg gggccgggcg cagtggctca cgaatcccac 18120
   actctgggag gccaaggcga gtggatcacc tgaggtcggg agtttgagcc cagcctgacc 18180
   aacatggaga aaccccatct ctactaaaaa tacaaaagta gccgggcgtg gtgatgcatg 18240
   cctgtaatcc cagctaccta ctcgggaggc tgaggcagga gaatcgcttg aacccgggag 18300
   gcagaggttg cggtgagctg agatctcacc attacactcc agcctgggca acaagagtga 18360
   aactccgtct caaaaaagta taataaagta aaatggggta agggaagatt acgagactaa 18420
   tacacactaa tactctgagg tgctcagtaa acatatttgc atggggtgtg gccaccatct 18480
   tgatttgaat tcccgttgtc ccagccttag gcccttcaaa gcattggtca gggaaaaggg 18540
   gcacagaccc tctcactcat gtgatgtcat ctctcctccc tgcttctgtc tcctacagcc 18600
   acctgaagtc caaaaagggt cagtctacct cccgccataa aaaactcatg ttcaagacag 18660
   aagggcctga ctcagactga cattctccac ttcttgttcc ccactgacag cctcccaccc 18720
   ccatctctcc ctcccctgcc attttgggtt ttgggtcttt gaacccttgc ttgcaatagg 18780
   tgtgcgtcag aagcacccag gacttccatt tgctttgtcc cggggctcca ctgaacaagt 18840
   tggcctgcac tggtgttttg ttgtggggag gaggatgggg agtaggacat accagcttag 18900
   attttaaggt ttttactgtg agggatgttt gggagatgta agaaatgttc ttgcagttaa 18960
   gggttagttt acaatcagcc acattctagg taggtagggg cccacttcac cgtactaacc 19020
   agggaagctg tccctcatgt tgaattttct ctaacttcaa ggcccatatc tgtgaaatgc 19080
   tggcatttgc acctacctca cagagtgcat tgtgagggtt aatgaaataa tgtacatctg 19140
   gccttgaaac caccttttat tacatggggt ctaaaacttg acccccttga gggtgcctgt 19200
   tccctctccc tctccctgtt ggctggtggg ttggtagttt ctacagttgg gcagctggtt 19260
   aggtagaggg agttgtcaag tcttgctggc ccagccaaac cctgtctgac aacctcttgg 19320
   tcgaccttag tacctaaaag gaaatctcac cccatcccac accctggagg atttcatctc 19380
   ttgtatatga tgatctggat ccaccaagac ttgttttatg ctcagggtca atttcttttt 19440
   tctttttttt tttttttttt ctttttcttt gagactgggt ctcgctttgt tgcccaggct 19500
   ggagtggagt ggcgtgatct tggcttactg cagcctttgc ctccccggct cgagcagtcc 19560
   tgcctcagcc tccggagtag ctgggaccac aggttcatgc caccatggcc agccaacttt 19620
   tgcatgtttt gtagagatgg ggtctcacag tgttgcccag gctggtctca aactcctggg 19680
   ctcaggcgat ccacctgtct cagcctccca gagtgctggg attacaattg tgagccacca 19740
   cgtggagctg gaagggtcaa catcttttac attctgcaag cacatctgca ttttcacccc 19800
   acccttcccc tccttctccc tttttatatc ccatttttat atcgatctct tattttacaa 19860
   taaaactttg ctgccacctg tgtgtctgag gggtgaacgc cagtgcaggc tactggggtc 19920
   agcaggtgca ggggtgagtg aggaggtgct gggaagcagc cacctgagtc tgcaatgagt 19980
   gtggactggg gggcccagtg cccgggttcc gggaggggaa caaaggctgg agactgggtc 20040
   agtctgcggg ctgcatgaca acaagggagg gggtggctcc attcataact caggaaccaa 20100
   ccgtccctcc tcccctccgc ccacggctgg cacaaggttc tctgcctccc ctgcttctag 20160
   gattgggctg cttccccctc ggcagcctct caccaaggat tacgggattt aaatgtcgtg 20220
   atttacgaag gctgagcctc cagggtggcc atcttcgtcc atcagaagtg gcaggatacc 20280
   tgggttccaa gggaacaggg tgg 20303
<210> 2
   <211> 36
   <212> DNA
   <213> artificial
<220>
   <223> primer
<220>
   <221> misc_feature
   <222> (1)..(36)
<400> 2
   gatcgatcga tcgatcttct ctgcaggccc aggtga 36
<210> 3
   <211> 20
   <212> DNA
   <213> artificial
<220>
   <223> primer
<220>
   <221> misc_feature
   <222> (1)..(20)
<400> 3
   ttctctgcag gcccaggtga 20
<210> 4
   <211> 21
   <212> DNA
   <213> artificial
<220>
   <223> primer
<220>
   <221> misc_feature
   <222> (1)..(21)
<400> 4
   tcgcttccca caggtctctg c 21
<210> 5
   <211> 20
   <212> DNA
   <213> artificial
<220>
   <223> primer
<220>
   <221> misc_feature
   <222> (1)..(20)
<400> 5
   aaccccagcc ccctagcaga 20
<210> 6
   <211> 20
   <212> DNA
   <213> artificial
<220>
   <223> primer
<220>
   <221> misc_feature
   <222> (1)..(20)
<400> 6
   ccggggacag catcaaatca 20
<210> 7
   <211> 19
   <212> DNA
   <213> artificial
<220>
   <223> primer
<220>
   <221> misc_feature
   <222> (1)..(19)
<400> 7
   agggttgggc tggggacct 19
<210> 8
   <211> 20
   <212> DNA
   <213> artificial
<220>
   <223> primer
<220>
   <221> misc_feature
   <222> (1)..(20)
<400> 8
   gggatacggc caggcattga 20
<210> 9
   <211> 27
   <212> DNA
   <213> artificial
<220>
   <223> primer
<220>
   <221> misc_feature
   <222> (1)..(27)
<400> 9
   ccagttgctt tatctgttca cttgtgc 27
<210> 10
   <211> 18
   <212> DNA
   <213> artificial
<220>
   <223> primer
<220>
   <221> misc_feature
   <222> (1)..(18)
<400> 10
   ctggggaccc tgggcaac 18
<210> 11
   <211> 20
   <212> DNA
   <213> artificial
<220>
   <223> primer
<220>
   <221> misc_feature
   <222> (1)..(20)
<400> 11
   agctggggct ggagagacga 20
<210> 12
   <211> 19
   <212> DNA
   <213> artificial
<220>
   <223> primer
<220>
   <221> misc_feature
   <222> (1)..(19)
<400> 12
   ccggagggcc actgacaac 19
<210> 13
   <211> 20
   <212> DNA
   <213> artificial
<220>
   <223> primer
<220>
   <221> misc_feature
   <222> (1)..(20)
<400> 13
   aaaaggcctc ccctgcttgc 20
<210> 14
   <211> 19
   <212> DNA
   <213> artificial
<220>
   <223> primer
<220>
   <221> misc_feature
   <222> (1)..(19)
<400> 14
   aagcagaggc tggggcaca 19
<210> 15
   <211> 24
   <212> DNA
   <213> artificial
<220>
   <223> primer
<220>
   <221> misc_feature
   <222> (1)..(24)
<400> 15
   tgggacaggt aggacctgat ttcc 24
<210> 16
   <211> 23
   <212> DNA
   <213> artificial
<220>
   <223> primer
<220>
   <221> misc_feature
   <222> (1)..(23)
<400> 16
   ggcataactg cacccttggt ctc 23
<210> 17
   <211> 20
   <212> DNA
   <213> artificial
<220>
   <223> primer
<220>
   <221> misc_feature
   <222> (1)..(20)
<400> 17
   agcggtggag gagaccaagg 20
<210> 18
   <211> 22
   <212> DNA
   <213> artificial
<220>
   <223> primer
<220>
   <221> misc_feature
   <222> (1)..(22)
<400> 18
   tgccccaatt gcaggtaaaa ca 22
<210> 19
   <211> 23
   <212> DNA
   <213> artificial
<220>
   <223> primer
<220>
   <221> misc_feature
   <222> (1)..(23)
<400> 19
   tcgatgttgc ttttgatccg tca 23
<210> 20
   <211> 25
   <212> DNA
   <213> artificial
<220>
   <223> primer
<220>
   <221> misc_feature
   <222> (1)..(25)
<400> 20
   aatggaatcc tatggctttc caacc 25
<210> 21
   <211> 20
   <212> DNA
   <213> artificial
<220>
   <223> primer
<220>
   <221> misc_feature
   <222> (1)..(20)
<400> 21
   ggtcagggaa aaggggcaca 20
<210> 22
   <211> 20
   <212> DNA
   <213> artificial
<220>
   <223> primer
<220>
   <221> misc_feature
   <222> (1)..(20)
<400> 22
   tggcagggga gggagagatg 20
<210> 23
   <211> 19
   <212> DNA
   <213> artificial
<220>
   <223> primer
<220>
   <221> misc_feature
   <222> (1)..(19)
<400> 23
   ctctgactgc tcttttcac 19
<210> 24
   <211> 20
   <212> DNA
   <213> artificial
<220>
   <223> primer
<220>
   <221> misc_feature
   <222> (1)..(20)
<400> 24
   cattgaagtc tcatggaagc 20